(19) Europäisches Patentamt
European Patent Office
Office européen des brevets

(11) EP 1 578 791 B1

(12) **EUROPEAN PATENT SPECIFICATION**

(45) Date of publication and mention of the grant of the patent:
**12.03.2008 Bulletin 2008/11**

(21) Application number: **03789657.8**

(22) Date of filing: **31.12.2003**

(51) Int Cl.:
*C07K 14/435* (2006.01) *C07K 14/02* (2006.01)
*C07K 19/00* (2006.01) *A61K 39/00* (2006.01)

(86) International application number:
**PCT/PL2003/000151**

(87) International publication number:
**WO 2004/058816 (15.07.2004 Gazette 2004/29)**

(54) **INCLUSION BODIES FOR THE ORAL VACCINATION OF ANIMALS**
EINSCHLUSSKÖRPER FÜR DIE MÜNDLICHE IMPFUNG VON TIEREN
CORPS D'INCLUSION POUR LA VACCINATION ORALE D'ANIMAUX

(84) Designated Contracting States:
**AT BE BG CH CY CZ DE DK EE ES FI FR GB GR HU IE IT LI LU MC NL PT RO SE SI SK TR**

(30) Priority: **31.12.2002 PL 2358081**

(43) Date of publication of application:
**28.09.2005 Bulletin 2005/39**

(73) Proprietors:
- **Instytut Biotechnologii I Antybiotykow 02-516 Warszawa (PL)**
- **Instytut Parazytologii Pan PL-00-818 Warszawa (PL)**

(72) Inventors:
- **PLUCIENNICZAK, Andrzej PL-04-360 Warszawa (PL)**
- **KESIK, Malgorzata PL-00-137 Warszawa (PL)**
- **POREBSKA, Anna PL-02-615 Warszawa (PL)**
- **WEDRYCHOWICZ, Halina PL-04-391 Warsawa (PL)**
- **JEDLINA-PANASIUK, Luiza PL-00-818 Warszawa (PL)**

(74) Representative: **Witek, Rafal WTS Patent Attorneys, ul. Rudolfa Weigla 12 53-114 Wroclaw (PL)**

(56) References cited:
**WO-A-94/17820** **WO-A-20/04050883**

- **CARNEVALE SILVANA ET AL: "Immunodiagnosis of fasciolosis using recombinant procathepsin L cystein proteinase" DIAGNOSTIC MICROBIOLOGY AND INFECTIOUS DISEASE, vol. 41, no. 1-2, September 2001 (2001-09), pages 43-49, XP001182394 ISSN: 0732-8893**
- **CORNELISSEN JAN B W J ET AL: "Early immunodiagnosis of fasciolosis in ruminants using recombinant Fasciola hepatica cathepsin L-like protease" INTERNATIONAL JOURNAL FOR PARASITOLOGY, vol. 31, no. 7, 15 May 2001 (2001-05-15), pages 728-737, XP002289120 ISSN: 0020-7519**
- **SPITHILL T W ET AL: "Progress in development of liver fluke vaccines" PARASITOLOGY TODAY, vol. 14, no. 6, June 1998 (1998-06), pages 224-228, XP002289121 ISSN: 0169-4758**
- **ROTHEL J S ET AL: "Urea/DTT solubilization of a recombinant Taenia ovis antigen, 45W, expressed as a GST fusion protein results in enhanced protective immune response to the 45W moiety" VACCINE, BUTTERWORTH SCIENTIFIC. GUILDFORD, GB, vol. 15, no. 5, 5 April 1997 (1997-04-05), pages 469-472, XP004059883 ISSN: 0264-410X**
- **WEDRYCHOWICZ H ET AL: "The immune response of rats to vaccination with the cDNA or protein forms of the cysteine proteinase of Fasciola hepatica." VETERINARY IMMUNOLOGY AND IMMUNOPATHOLOGY, vol. 94, no. 1-2, 15 July 2003 (2003-07-15), pages 83-93, XP002289122 ISSN: 0165-2427**

EP 1 578 791 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **KESIK MALGORZATA ET AL: "Inclusion bodies from recombinant bacteria as a novel system for delivery of vaccine antigen by the oral route." IMMUNOLOGY LETTERS, vol. 91, no. 2-3, 15 February 2004 (2004-02-15), pages 197-204, XP002289123 ISSN: 0165-2478**

SEQUENCE LISTING

<110> INSTYTUT BIOTECHNOLOGII I ANTYBIOTYKÓW INSTYTUT PARAZYTOLOGII PAN

<120> Inclusion bodies for the oral vaccination of animals

<130> PZ/0060/RW

<150> PL358081
<151> 2002-12-31

<160> 10

<170> PatentIn version 3.2

<210> 1
<211> 991

<212> DNA
<213> Fasciola hepatica

<400> 1

```
ggatccatgt ggttcttcgt attagccgtc ctcacagtcg gagtgcttgg ctcgaatgat     60
gatttgtggc atcagtggaa gcgaatgtac aacaatgaat acaatggggc tgacgatcag    120
cacagacgaa atatttggga agagaatgtg aaacatatcc aagaacataa cctacgtcac    180
gatctcggcc tcgtcaccta cacattggga ttgaaccaat tcacggatat gacattcgag    240
gaattcaagg ccaaatatct aacagaaatg tcacgcgcgt ccgatatact ctcacacggt    300
gtcccgtatg agacgaacaa tcgtgccgta cccgacaaaa ttgactggcg tgaatctggt    360
tatgtgacgg aggtgaaaga tcagggaaac tgtggttcct gttgggcatt ctcaacaacc    420
ggtactatgg agggacagta tatgaaaaac gaaagaacta gtatttcatt ctctgagcaa    480
caactggtcg attgtagcgg tccttgggga aataatggtt gcagtggtgg attgatggaa    540
aatgcttacc aatatttgaa acaatttgga ttggaaaccg aatcctctta tccgtacacg    600
gctgtggaag gtcagtgtcg atacaataag cagttaggag ttgccaaagt gactggctac    660
tacactgtgc cttctggcag tgaggtagaa ttgaaaaatc tagtcggtgc cgaaggacct    720
gccgcggtcg ctgtggatgt ggaatctgac ttcatgatgt acaggagtgg tatttatcag    780
agccaaactt gttcaccgct tcgtgtgaat catgcagtct tggctgtcgg ttacggaaca    840
cagggtggta ctgactattg gattgtgaaa aatagttggg gattgtcgtg gggtgagcgc    900
ggttacattc gaatggctag gaatcgaggt aacatgtgtg gaattgcttc gctggccagt    960
ctcccgattg gcacgatttc cgtgactcga g                                   991
```

<210> 2
<211> 1198
<212> DNA
<213> synthetic

<400> 2

```
catatggata tcgatcctta taaagaattt ggagctactg tggagttact ctcgtttttg     60
ccttctgact tctttccttc agtacgagat cttctggata ccgcctcagc tctctatcgg    120
gaagccttag agtctcctga gcattgttca cctcaccata ctgcactcag gcaagcaatt    180
ctttgctggg gggaactaat gactctagct acctgggtgg gtgttaatct cgaggatcaa    240
ggtggtggag gttctggtgg aggaggccaa ggaggcgctg tacccgacaa aattgactgg    300
cgtgaatctg gttatgtgac ggaggtgaaa gatcagggaa actgtggttc ctgttgggca    360
ttctcaacaa ccggtactat ggagggacag tatatgaaaa acgaaagaac tagtatttca    420
ttctctgagc aacaactggt cgattgtagc ggtccttggg gaaataatgg ttgcagtggt    480
ggattgatgg aaaatgctta ccaatatttg aaacaatttg gattggaaac cgaatcctct    540
tatccgtaca cggctgtgga aggtcagtgt cgatacaata agcagttagg agttgccaaa    600
gtgactggct actacactgt gccttctggc agtgaggtag aattgaaaaa tctagtcggt    660
gccgaaggac ctgccgcggt cgctgtggat gtggaatctg acttcatgat gtacaggagt    720
ggtatttatc agagccaaac ttgttcaccg cttcgtgtga atcatgcagt cttggctgtc    780
ggttacggaa cacagggtgg tactgactat tggattgtga aaaatagttg gggattgtcg    840
tggggtgagc gcggttacat tcgaatggct aggaatcgag gtaacatgtg tggaattgct    900
tcgctggcca gtctcccgat tggcacgatt tccggcatcg atggccaagg aggccaagga    960
ggaggtggtt caggcggtgg tggatctagg gacctagttg ttagttatgt caacactaat   1020
atgggcttaa agttcaggca actttgtgg tttcacattt cttgtctcac ttttggaaga    1080
gaaacggtca tagagtattt ggtgtctttc ggagtgtgga ttcgcactcc tccagcttat   1140
agaccaccaa atgcccctat cttatcaaca cttccggaga ctactgttgt ttaagctt     1198
```

<210> 3
<211> 1198
<212> DNA
<213> synthetic

<400> 3

```
catatggata tcgatcctta taaagaattt ggagctactg tggagttact ctcgtttttg    60
ccttctgact tctttccttc agtacgagat cttctggata ccgcctcagc tctctatcgg   120
gaagccttag agtctcctga gcattgttca cctcaccata ctgcactcag gcaagcaatt   180
ctttgctggg gggaactaat gactctagct acctgggtgg gtgttaatct cgaggatcaa   240
ggtggtggag gttctggtgg aggaggccaa ggaggcgctg tacccgacaa aattgactgg   300
cgtgaatctg gttatgtgac ggaggtgaaa gatcagggaa actgtggttc ctgttgggca   360
ttctcaacaa ccggtactat ggagggacag tatatgaaaa acgaaagaac tagtatttca   420
ttctctgagc aacaactggt cgattgtagc ggtccttggg gaaataatgg ttgcagtggt   480
ggattgatgg aaaatgctta ccaatatttg aaacaatttg gattggaaac cgaatcctct   540
tatccgtaca cggctgtgga aggtcagtgt cgatacaata agcagttagg agttgccaaa   600
gtgactggct actacactgt gccttctggc agtgaggtag aattgaaaaa tctagtcggt   660
gccgaaggac ctgccgcggt cgctgtggat gtggaatctg acttcatgat gtacaggagt   720
ggtatttatc agagccaaac ttgttcaccg cttcgtgtga atcatgcagt cttggctgtc   780
ggttacggaa cacagggtgg tactgactat tggattgtga aaaatagttg gggattgtcg   840
tggggtgagc gcggttacat tcgaatggct aggaatcgag gtaacatgtg tggaattgct   900
tcgctggcca gtctcccgat tggcacgatt tccggcatcg atggccaagg aggccaagga   960
ggaggtggtt caggcggtgg tggatctagg gacctagttg ttagttatgt caacactaat  1020
atgggcttaa agttcaggca acttttgtgg tttcacattt cttgtctcac ttttggaaga  1080
gaaacggtca tagagtattt ggtgtctttc ggagtgtgga ttcgcactcc tccagcttat  1140
agaccaccaa atgcccctat cttatcaaca cttccggaga ctactgttgt ttaagctt    1198
```

<210> 4
<211> 1127
<212> DNA
<213> synthetic

<400> 4

```
ggatccatat ggctgtaccc gacaaaattg actggcgtga atctggttat gtgacggagg    60
tgaaagatca gggaaactgt ggttcctgtt gggcattctc aacaaccggt actatggagg   120
gacagtatat gaaaaacgaa agaactagta tttcattctc tgagcaacaa ctggtcgatt   180
gtagcggtcc ttggggaaat aatggttgca gtggtggatt gatggaaaat gcttaccaat   240
atttgaaaca atttggattg gaaaccgaat cctcttatcc gtacacggct gtggaaggtc   300
agtgtcgata caataagcag ttaggagttg ccaaagtgac tggctactac actgtgcctt   360
ctggcagtga ggtagaattg aaaaatctag tcggtgccga aggacctgcc gcggtcgctg   420
tggatgtgga atctgacttc atgatgtaca ggagtggtat ttatcagagc caaacttgtt   480
caccgcttcg tgtgaatcat gcagtcttgg ctgtcggtta cggaacacag ggtggtactg   540
actattggat tgtgaaaaat agttggggat tgtcgtgggg tgagcgcggt tacattcgaa   600
tggctaggaa tcgaggtaac atgtgtggaa ttgcttcgct ggccagtctc ccgattggca   660
cgatttccgg catcgatcct tataaagaat ttggagctac tgtggagtta ctctcgtttt   720
tgccttctga cttctttcct tcagtacgag atcttctaga taccgcctca gctctctatc   780
gggaagcctt agagtctcct gagcattgtt cacctcacca tactgcactc aggcaagcaa   840
ttctttgctg gggggaacta atgactctag ctacctgggt gggtgttaat ttggaagatc   900
cagcatctag ggacctagtt gttagttatg tcaacactaa tatgggctta aagttcaggc   960
aacttttgtg gtttcacatt tcttgtctca cttttggaag agaaacggtc atagagtatt  1020
tggtgtcttt cggagtgtgg attcgcactc ctccagctta tagaccacca aatgccccta  1080
tcttatcaac acttccggag actactgttg tttaaaagct tgagctc                1127
```

<210> 5
<211> 699
<212> DNA
<213> synthetic

<400> 5

```
ggatccatat ggctgtaccc gacaaaattg actggcgtga atctggttat gtgacggagg      60
tgaaagatca gggaaactgt ggttcctgtt gggcattctc aacaaccggt actatggagg     120
gacagtatat gaaaaacgaa agaactagta tttcattctc tgagcaacaa ctggtcgatt     180
gtagcggtcc ttggggaaat aatggttgca gtggtggatt gatggaaaat gcttaccaat     240
atttgaaaca atttggattg gaaaccgaat cctcttatcc gtacacggct gtggaaggtc     300
agtgtcgata caataagcag ttaggagttg ccaaagtgac tggctactac actgtgcctt     360
```

```
ctggcagtga ggtagaattg aaaaatctag tcggtgccga aggacctgcc gcggtcgctg     420
tggatgtgga atctgacttc atgatgtaca ggagtggtat ttatcagagc caaacttgtt     480
caccgcttcg tgtgaatcat gcagtcttgg ctgtcggtta cggaacacag ggtggtactg     540
actattggat tgtgaaaaat agttggggat tgtcgtgggg tgagcgcggt tacattcgaa     600
tggctaggaa tcgaggtaac atgtgtggaa ttgcttcgct ggccagtctc ccgatggtgg     660
cacgatttcc gggtagcggt agtgatatcg attaagctt                            699
```

<210> 6
<211> 694
<212> DNA
<213> Fasciola hepatica

<400> 6

```
catatggctg tacccgacaa aattgactgg cgtgaatctg gttatgtgac ggaggtgaaa      60
gatcagggaa actgtggttc ctgttgggca ttctcaacaa ccggtactat ggagggacag     120
tatatgaaaa acgaaagaac tagtatttca ttctctgagc aacaactggt cgattgtagc     180
ggtccttggg gaaataatgg ttgcagtggt ggattgatgg aaaatgctta ccaatatttg     240
aaacaatttg gattggaaac cgaatcctct tatccgtaca cggctgtgga aggtcagtgt     300
cgatacaata agcagttagg agttgccaaa gtgactggct actacactgt gccttctggc     360
agtgaggtag aattgaaaaa tctagtcggt gccgaaggac ctgccgcggt cgctgtggat     420
gtggaatctg acttcatgat gtacaggagt ggtatttatc agagccaaac ttgttcaccg     480
cttcgtgtga atcatgcagt cttggctgtc ggttacggaa cacagggtgg tactgactat     540
tggattgtga aaaatagttg gggattgtcg tggggtgagc gcggttacat tcgaatggct     600
aggaatcgag gtaacatgtg tggaattgct tcgctggcca gtctcccgat ggtggcacga     660
tttccgggta gcggtagtga tatcgattaa gctt                                 694
```

<210> 7
<211> 686
<212> DNA
<213> synthetic

<400> 7

```
ggatccatat ggctgtaccc gacaaaattg actggcgtga atctggttat gtgacggagg      60
tgaaagatca gggaaactgt ggttcctgtt gggcattctc aacaaccggt actatggagg     120
gacagtatat gaaaaacgaa agaactagta tttcattctc tgagcaacaa ctggtcgatt     180
gtagtggtcc ttggggaaat aatggttgca gtggtggatt gatggaaaat gcttaccaat     240
atttgaaaca atttggattg gaaaccgaat cctcttatcc gtacacggct gtggaaggtc     300
agtgtcgata caataagcag ttaggagttg ccaaagtgac tggctactac actgtgcctt     360
ctggcagtga ggtagaattg aaaaatctag tcggtgccga aggacctgcc gcggtcgctg     420
tggatgtgga atctgacttc atgatgtaca ggagtggtat ttatcagagc caaacttgtt     480
caccgcttcg tgtgaatcat gcagtcttgg ctgtcggtta cggaacacag ggtggtactg     540
actattggat tgtgaaaaat agttggggat tgtcgtgggg tgagcgcggt tacattcgaa     600
tggctaggaa tcgaggtaac atgtgtggaa ttgcttcgct ggccagtctc ccgatggtgg     660
cacgatttcc ggcatcgatt aagctt                                          686
```

<210> 8
<211> 681
<212> DNA
<213> synthetic

<400> 8

```
catatggctg tacccgacaa aattgactgg cgtgaatctg gttatgtgac ggaggtgaaa     60
gatcagggaa actgtggttc ctgttgggca ttctcaacaa ccggtactat ggagggacag    120
tatatgaaaa acgaaagaac tagtatttca ttctctgagc aacaactggt cgattgtagt    180
ggtccttggg gaaataatgg ttgcagtggt ggattgatgg aaaatgctta ccaatatttg    240
aaacaatttg gattggaaac cgaatcctct tatccgtaca cggctgtgga aggtcagtgt    300
cgatacaata agcagttagg agttgccaaa gtgactggct actacactgt gccttctggc    360
agtgaggtag aattgaaaaa tctagtcggt gccgaaggac ctgccgcggt cgctgtggat    420
gtggaatctg acttcatgat gtacaggagt ggtatttatc agagccaaac ttgttcaccg    480
cttcgtgtga atcatgcagt cttggctgtc ggttacggaa cacagggtgg tactgactat    540
tggattgtga aaaatagttg ggattgtcg tggggtgagc gcggttacat tcgaatggct     600
aggaatcgag gtaacatgtg tggaattgct tcgctggcca gtctcccgat ggtggcacga    660
tttccggcat cgattaagct t                                              681
```

<210> 9
<211> 1116
<212> DNA
<213> synthetic

<400> 9

```
catatggctg tacccgacaa aattgactgg cgtgaatctg gttatgtgac ggaggtgaaa     60
gatcagggaa actgtggttc ctgttgggca ttctcaacaa ccggtactat ggagggacag    120
tatatgaaaa acgaaagaac tagtatttca ttctctgagc aacaactggt cgattgtagc    180
ggtccttggg gaaataatgg ttgcagtggt ggattgatgg aaaatgctta ccaatatttg    240
aaacaatttg gattggaaac cgaatcctct tatccgtaca cggctgtgga aggtcagtgt    300
cgatacaata agcagttagg agttgccaaa gtgactggct actacactgt gccttctggc    360
agtgaggtag aattgaaaaa tctagtcggt gccgaaggac ctgccgcggt cgctgtggat    420
gtggaatctg acttcatgat gtacaggagt ggtatttatc agagccaaac ttgttcaccg    480
cttcgtgtga atcatgcagt cttggctgtc ggttacggaa cacagggtgg tactgactat    540
tggattgtga aaaatagttg ggattgtcg tggggtgagc gcggttacat tcgaatggct     600
aggaatcgag gtaacatgtg tggaattgct tcgctggcca gtctcccgat tggcacgatt    660
tccggcatcg atccttataa agaatttgga gctactgtgg agttactctc gtttttgcct    720
tctgacttct ttccttcagt acgagatctt ctagataccg cctcagctct ctatcgggaa    780
gccttagagt ctcctgagca ttgttcacct caccatactg cactcaggca agcaattctt    840
tgctgggggg aactaatgac tctagctacc tgggtgggtg ttaatttgga agatccagca    900
tctagggacc tagttgttag ttatgtcaac actaatatgg gcttaaagtt caggcaactt    960
ttgtggtttc acatttcttg tctcactttt ggaagagaaa cggtcataga gtatttggtg   1020
tctttcggag tgtggattcg cactcctcca gcttatagac caccaaatgc ccctatctta   1080
tcaacacttc cggagactac tgttgtttaa aagctt                             1116
```

<210> 10
<211> 952
<212> DNA
<213> synthetic

<400> 10

```
catatgtcga atgatgattt gtggcatcag tggaagcgaa tgtacaacaa tgaatacaat      60
ggggctgacg atcagcacag acgaaatatt tgggaagaga atgtgaaaca tatccaagaa     120
cataacctac gtcacgatct cggcctcgtc acctacacat tgggattgaa ccaattcacg     180
gatatgacat tcgaggaatt caaggccaaa tatctaacag aaatgtcacg cgcgtccgat     240
atactctcac acggtgtccc gtatgagacg aacaatcgtg ccgtacccga caaaattgac     300
tggcgtgaat ctggttatgt gacggaggtg aaagatcagg gaaactgtgg ttcctgttgg     360
gcattctcaa caaccggtac tatggaggga cagtatatga aaaacgaaag aactagtatt     420
tcattctctg agcaacaact ggtcgattgt agcggtcctt ggggaaataa tggttgcagt     480
ggtggattga tggaaaatgc ttaccaatat ttgaaacaat ttggattgga aaccgaatcc     540
tcttatccgt acacggctgt ggaaggtcag tgtcgataca ataagcagtt aggagttgcc     600
aaagtgactg gctactacac tgtgccttct ggcagtgagg tagaattgaa aaatctagtc     660
ggtgccgaag gacctgccgc ggtcgctgtg gatgtggaat ctgacttcat gatgtacagg     720
agtggtattt atcagagcca aacttgttca ccgcttcgtg tgaatcatgc agtcttggct     780
gtcggttacg gaacacaggg tggtactgac tattggattg tgaaaaatag ttggggattg     840
tcgtggggtg agcgcggtta cattcgaatg gctaggaatc gaggtaacat gtgtggaatt     900
gcttcgctgg ccagtctccc gattggcacg atttccggca tcgattaagc tt             952
```

SEQUENCE LISTING

<110> INSTYTUT BIOTECHNOLOGII I ANTYBIOTYKÓW INSTYTUT PARAZYTOLOGII PAN
<120> Inclusion bodies for the oral vaccination of animals
<130> PZ/0060/RW
<140> PCT/PL03/00151
<141> 2003-12-31
<150> PL358081
<151> 2002-12-31
<160> 17
<170> PatentIn version 3.1
<210> 1
<211> 991
<212> DNA
<213> Fasciola hepatica
<220>
<221> gene
<222> (1)..(991)
<223>
<220>
<221> gene
<222> (1)..(991)
<223> liver fluke cysteine protease
<400> 1

```
ggatccatgt ggttcttcgt attagccgtc ctcacagtcg gagtgcttgg ctcgaatgat     60
gatttgtggc atcagtggaa gcgaatgtac aacaatgaat acaatggggc tgacgatcag    120
cacagacgaa atatttggga agagaatgtg aaacatatcc aagaacataa cctacgtcac    180
gatctcggcc tcgtcaccta cacattggga ttgaaccaat tcacggatat gacattcgag    240
gaattcaagg ccaaatatct aacagaaatg tcacgcgcgt ccgatatact ctcacacggt    300
gtcccgtatg agacgaacaa tcgtgccgta cccgacaaaa ttgactggcg tgaatctggt    360
tatgtgacgg aggtgaaaga tcagggaaac tgtggttcct gttgggcatt ctcaacaacc    420
ggtactatgg agggacagta tatgaaaaac gaaagaacta gtatttcatt ctctgagcaa    480
caactggtcg attgtagcgg tccttgggga aataatggtt gcagtggtgg attgatggaa    540
aatgcttacc aatatttgaa acaatttgga ttggaaaccg aatcctctta tccgtacacg    600
gctgtggaag gtcagtgtcg atacaataag cagttaggag ttgccaaagt gactggctac    660
tacactgtgc cttctggcag tgaggtagaa ttgaaaaatc tagtcggtgc cgaaggacct    720
gccgcggtcg ctgtggatgt ggaatctgac ttcatgatgt acaggagtgg tatttatcag    780
agccaaactt gttcaccgct tcgtgtgaat catgcagtct tggctgtcgg ttacggaaca    840
cagggtggta ctgactattg gattgtgaaa aatagttggg gattgtcgtg gggtgagcgc    900
ggttacattc gaatggctag gaatcgaggt aacatgtgtg gaattgcttc gctggccagt    960
ctcccgattg gcacgatttc cgtgactcga g                                   991
```

<210> 2
<211> 1198
<212> DNA
<213> artificial sequence
<220>
<223> CWekGlyKatwBluescr
<220>
<221> CDS
<222> (4)..(1194)
<223> sequence coding the hybrid protein HBV core domain:: catalytic do main of the liver fluke cysteine protease
<400> 2

```
cat atg gat atc gat cct tat aaa gaa ttt gga gct act gtg gag tta      48
    Met Asp Ile Asp Pro Tyr Lys Glu Phe Gly Ala Thr Val Glu Leu
    1               5                   10                  15
ctc tcg ttt ttg cct tct gac ttc ttt cct tca gta cga gat ctt ctg      96
Leu Ser Phe Leu Pro Ser Asp Phe Phe Pro Ser Val Arg Asp Leu Leu
                20                  25                  30
gat acc gcc tca gct ctc tat cgg gaa gcc tta gag tct cct gag cat     144
```

```
Asp Thr Ala Ser Ala Leu Tyr Arg Glu Ala Leu Glu Ser Pro Glu His
            35                  40                  45
tgt tca cct cac cat act gca ctc agg caa gca att ctt tgc tgg ggg      192
Cys Ser Pro His His Thr Ala Leu Arg Gln Ala Ile Leu Cys Trp Gly
        50                  55                  60
gaa cta atg act cta gct acc tgg gtg ggt gtt aat ctc gag gat caa      240
Glu Leu Met Thr Leu Ala Thr Trp Val Gly Val Asn Leu Glu Asp Gln
    65                  70                  75
ggt ggt gga ggt tct ggt gga gga ggc caa gga ggc gct gta ccc gac      288
Gly Gly Gly Gly Ser Gly Gly Gly Gly Gln Gly Gly Ala Val Pro Asp
80                  85                  90                  95
aaa att gac tgg cgt gaa tct ggt tat gtg acg gag gtg aaa gat cag      336
Lys Ile Asp Trp Arg Glu Ser Gly Tyr Val Thr Glu Val Lys Asp Gln
                100                 105                 110
gga aac tgt ggt tcc tgt tgg gca ttc tca aca acc ggt act atg gag      384
Gly Asn Cys Gly Ser Cys Trp Ala Phe Ser Thr Thr Gly Thr Met Glu
            115                 120                 125
gga cag tat atg aaa aac gaa aga act agt att tca ttc tct gag caa      432
Gly Gln Tyr Met Lys Asn Glu Arg Thr Ser Ile Ser Phe Ser Glu Gln
        130                 135                 140
caa ctg gtc gat tgt agc ggt cct tgg gga aat aat ggt tgc agt ggt      480
Gln Leu Val Asp Cys Ser Gly Pro Trp Gly Asn Asn Gly Cys Ser Gly
    145                 150                 155
gga ttg atg gaa aat gct tac caa tat ttg aaa caa ttt gga ttg gaa      528
Gly Leu Met Glu Asn Ala Tyr Gln Tyr Leu Lys Gln Phe Gly Leu Glu
160                 165                 170                 175
acc gaa tcc tct tat ccg tac acg gct gtg gaa ggt cag tgt cga tac      576
Thr Glu Ser Ser Tyr Pro Tyr Thr Ala Val Glu Gly Gln Cys Arg Tyr
                180                 185                 190
aat aag cag tta gga gtt gcc aaa gtg act ggc tac tac act gtg cct      624
Asn Lys Gln Leu Gly Val Ala Lys Val Thr Gly Tyr Tyr Thr Val Pro
            195                 200                 205
tct ggc agt gag gta gaa ttg aaa aat cta gtc ggt gcc gaa gga cct      672
Ser Gly Ser Glu Val Glu Leu Lys Asn Leu Val Gly Ala Glu Gly Pro
        210                 215                 220
gcc gcg gtc gct gtg gat gtg gaa tct gac ttc atg atg tac agg agt      720
Ala Ala Val Ala Val Asp Val Glu Ser Asp Phe Met Met Tyr Arg Ser
    225                 230                 235
ggt att tat cag agc caa act tgt tca ccg ctt cgt gtg aat cat gca      768
Gly Ile Tyr Gln Ser Gln Thr Cys Ser Pro Leu Arg Val Asn His Ala
240                 245                 250                 255
gtc ttg gct gtc ggt tac gga aca cag ggt ggt act gac tat tgg att      816
Val Leu Ala Val Gly Tyr Gly Thr Gln Gly Gly Thr Asp Tyr Trp Ile
                260                 265                 270
gtg aaa aat agt tgg gga ttg tcg tgg ggt gag cgc ggt tac att cga      864
Val Lys Asn Ser Trp Gly Leu Ser Trp Gly Glu Arg Gly Tyr Ile Arg
            275                 280                 285
atg gct agg aat cga ggt aac atg tgt gga att gct tcg ctg gcc agt      912
Met Ala Arg Asn Arg Gly Asn Met Cys Gly Ile Ala Ser Leu Ala Ser
        290                 295                 300
ctc ccg att ggc acg att tcc ggc atc gat ggc caa gga ggc caa gga      960
Leu Pro Ile Gly Thr Ile Ser Gly Ile Asp Gly Gln Gly Gly Gln Gly
    305                 310                 315
gga ggt ggt tca ggc ggt ggt gga tct agg gac cta gtt gtt agt tat     1008
Gly Gly Gly Ser Gly Gly Gly Gly Ser Arg Asp Leu Val Val Ser Tyr
320                 325                 330                 335
gtc aac act aat atg ggc tta aag ttc agg caa ctt ttg tgg ttt cac     1056
Val Asn Thr Asn Met Gly Leu Lys Phe Arg Gln Leu Leu Trp Phe His
                340                 345                 350
att tct tgt ctc act ttt gga aga gaa acg gtc ata gag tat ttg gtg     1104
Ile Ser Cys Leu Thr Phe Gly Arg Glu Thr Val Ile Glu Tyr Leu Val
```

```
            355                 360                 365
tct ttc gga gtg tgg att cgc act cct cca gct tat aga cca cca aat      1152
Ser Phe Gly Val Trp Ile Arg Thr Pro Pro Ala Tyr Arg Pro Pro Asn
        370                 375                 380
gcc cct atc tta tca aca ctt ccg gag act act gtt gtt taa gctt         1198
Ala Pro Ile Leu Ser Thr Leu Pro Glu Thr Thr Val Val
    385                 390                 395
```

<210> 3
<211> 396
<212> PRT
<213> artificial sequence
<220>
<223> CWekGlyKatwBluescr
<400> 3

```
Met Asp Ile Asp Pro Tyr Lys Glu Phe Gly Ala Thr Val Glu Leu Leu
1               5                   10                  15
Ser Phe Leu Pro Ser Asp Phe Phe Pro Ser Val Arg Asp Leu Leu Asp
            20                  25                  30
Thr Ala Ser Ala Leu Tyr Arg Glu Ala Leu Glu Ser Pro Glu His Cys
        35                  40                  45
Ser Pro His His Thr Ala Leu Arg Gln Ala Ile Leu Cys Trp Gly Glu
    50                  55                  60
Leu Met Thr Leu Ala Thr Trp Val Gly Val Asn Leu Glu Asp Gln Gly
65                  70                  75                  80
Gly Gly Gly Ser Gly Gly Gly Gly Gln Gly Gly Ala Val Pro Asp Lys
                85                  90                  95
Ile Asp Trp Arg Glu Ser Gly Tyr Val Thr Glu Val Lys Asp Gln Gly
            100                 105                 110
Asn Cys Gly Ser Cys Trp Ala Phe Ser Thr Thr Gly Thr Met Glu Gly
        115                 120                 125
Gln Tyr Met Lys Asn Glu Arg Thr Ser Ile Ser Phe Ser Glu Gln Gln
    130                 135                 140
Leu Val Asp Cys Ser Gly Pro Trp Gly Asn Asn Gly Cys Ser Gly Gly
145                 150                 155                 160
Leu Met Glu Asn Ala Tyr Gln Tyr Leu Lys Gln Phe Gly Leu Glu Thr
                165                 170                 175
Glu Ser Ser Tyr Pro Tyr Thr Ala Val Glu Gly Gln Cys Arg Tyr Asn
            180                 185                 190
Lys Gln Leu Gly Val Ala Lys Val Thr Gly Tyr Tyr Thr Val Pro Ser
        195                 200                 205
Gly Ser Glu Val Glu Leu Lys Asn Leu Val Gly Ala Glu Gly Pro Ala
    210                 215                 220
Ala Val Ala Val Asp Val Glu Ser Asp Phe Met Met Tyr Arg Ser Gly
225                 230                 235                 240
Ile Tyr Gln Ser Gln Thr Cys Ser Pro Leu Arg Val Asn His Ala Val
                245                 250                 255
Leu Ala Val Gly Tyr Gly Thr Gln Gly Gly Thr Asp Tyr Trp Ile Val
            260                 265                 270
Lys Asn Ser Trp Gly Leu Ser Trp Gly Glu Arg Gly Tyr Ile Arg Met
        275                 280                 285
Ala Arg Asn Arg Gly Asn Met Cys Gly Ile Ala Ser Leu Ala Ser Leu
    290                 295                 300
Pro Ile Gly Thr Ile Ser Gly Ile Asp Gly Gln Gly Gly Gln Gly Gly
305                 310                 315                 320
Gly Gly Ser Gly Gly Gly Gly Ser Arg Asp Leu Val Val Ser Tyr Val
                325                 330                 335
Asn Thr Asn Met Gly Leu Lys Phe Arg Gln Leu Leu Trp Phe His Ile
            340                 345                 350
Ser Cys Leu Thr Phe Gly Arg Glu Thr Val Ile Glu Tyr Leu Val Ser
        355                 360                 365
Phe Gly Val Trp Ile Arg Thr Pro Pro Ala Tyr Arg Pro Pro Asn Ala


    370                 375                 380
Pro Ile Leu Ser Thr Leu Pro Glu Thr Thr Val Val
385                 390                 395
```

<210> 4
<211> 1198
<212> DNA
<213> Artificial sequence
<220>
<223> CWekGlyKatwPIG

<220>
<221> CDS
<222> (4)..(1194)
<223> sequence coding the hybrid protein HBV core domain::catalytic dom ain of the liver fluke cysteine protease
<400> 4

```
cat atg gat atc gat cct tat aaa gaa ttt gga gct act gtg gag tta       48
    Met Asp Ile Asp Pro Tyr Lys Glu Phe Gly Ala Thr Val Glu Leu
    1               5                   10                  15
ctc tcg ttt ttg cct tct gac ttc ttt cct tca gta cga gat ctt ctg       96
Leu Ser Phe Leu Pro Ser Asp Phe Phe Pro Ser Val Arg Asp Leu Leu
                20                  25                  30
gat acc gcc tca gct ctc tat cgg gaa gcc tta gag tct cct gag cat      144
Asp Thr Ala Ser Ala Leu Tyr Arg Glu Ala Leu Glu Ser Pro Glu His
            35                  40                  45
tgt tca cct cac cat act gca ctc agg caa gca att ctt tgc tgg ggg      192
Cys Ser Pro His His Thr Ala Leu Arg Gln Ala Ile Leu Cys Trp Gly
        50                  55                  60
gaa cta atg act cta gct acc tgg gtg ggt gtt aat ctc gag gat caa      240
Glu Leu Met Thr Leu Ala Thr Trp Val Gly Val Asn Leu Glu Asp Gln
    65                  70                  75
ggt ggt gga ggt tct ggt gga gga ggc caa gga ggc gct gta ccc gac      288
Gly Gly Gly Gly Ser Gly Gly Gly Gly Gln Gly Gly Ala Val Pro Asp
80                  85                  90                  95
aaa att gac tgg cgt gaa tct ggt tat gtg acg gag gtg aaa gat cag      336
Lys Ile Asp Trp Arg Glu Ser Gly Tyr Val Thr Glu Val Lys Asp Gln
                100                 105                 110
gga aac tgt ggt tcc tgt tgg gca ttc tca aca acc ggt act atg gag      384
Gly Asn Cys Gly Ser Cys Trp Ala Phe Ser Thr Thr Gly Thr Met Glu
            115                 120                 125
gga cag tat atg aaa aac gaa aga act agt att tca ttc tct gag caa      432
Gly Gln Tyr Met Lys Asn Glu Arg Thr Ser Ile Ser Phe Ser Glu Gln
        130                 135                 140
caa ctg gtc gat tgt agc ggt cct tgg gga aat aat ggt tgc agt ggt      480
Gln Leu Val Asp Cys Ser Gly Pro Trp Gly Asn Asn Gly Cys Ser Gly
    145                 150                 155
gga ttg atg gaa aat gct tac caa tat ttg aaa caa ttt gga ttg gaa      528
Gly Leu Met Glu Asn Ala Tyr Gln Tyr Leu Lys Gln Phe Gly Leu Glu
160                 165                 170                 175
acc gaa tcc tct tat ccg tac acg gct gtg gaa ggt cag tgt cga tac      576
Thr Glu Ser Ser Tyr Pro Tyr Thr Ala Val Glu Gly Gln Cys Arg Tyr
                180                 185                 190
aat aag cag tta gga gtt gcc aaa gtg act ggc tac tac act gtg cct      624
Asn Lys Gln Leu Gly Val Ala Lys Val Thr Gly Tyr Tyr Thr Val Pro
            195                 200                 205
tct ggc agt gag gta gaa ttg aaa aat cta gtc ggt gcc gaa gga cct      672
Ser Gly Ser Glu Val Glu Leu Lys Asn Leu Val Gly Ala Glu Gly Pro
        210                 215                 220
gcc gcg gtc gct gtg gat gtg gaa tct gac ttc atg atg tac agg agt      720
Ala Ala Val Ala Val Asp Val Glu Ser Asp Phe Met Met Tyr Arg Ser
    225                 230                 235
```

```
ggt att tat cag agc caa act tgt tca ccg ctt cgt gtg aat cat gca        768
Gly Ile Tyr Gln Ser Gln Thr Cys Ser Pro Leu Arg Val Asn His Ala
240                 245                 250                 255
gtc ttg gct gtc ggt tac gga aca cag ggt ggt act gac tat tgg att        816
Val Leu Ala Val Gly Tyr Gly Thr Gln Gly Gly Thr Asp Tyr Trp Ile
                260                 265                 270
gtg aaa aat agt tgg gga ttg tcg tgg ggt gag cgc ggt tac att cga        864
Val Lys Asn Ser Trp Gly Leu Ser Trp Gly Glu Arg Gly Tyr Ile Arg
            275                 280                 285
atg gct agg aat cga ggt aac atg tgt gga att gct tcg ctg gcc agt        912
Met Ala Arg Asn Arg Gly Asn Met Cys Gly Ile Ala Ser Leu Ala Ser
        290                 295                 300
ctc ccg att ggc acg att tcc ggc atc gat ggc caa gga ggc caa gga        960
Leu Pro Ile Gly Thr Ile Ser Gly Ile Asp Gly Gln Gly Gly Gln Gly
    305                 310                 315
gga ggt ggt tca ggc ggt ggt gga tct agg gac cta gtt gtt agt tat       1008
Gly Gly Gly Ser Gly Gly Gly Gly Ser Arg Asp Leu Val Val Ser Tyr
320                 325                 330                 335
gtc aac act aat atg ggc tta aag ttc agg caa ctt ttg tgg ttt cac       1056
Val Asn Thr Asn Met Gly Leu Lys Phe Arg Gln Leu Leu Trp Phe His
                340                 345                 350
att tct tgt ctc act ttt gga aga gaa acg gtc ata gag tat ttg gtg       1104
Ile Ser Cys Leu Thr Phe Gly Arg Glu Thr Val Ile Glu Tyr Leu Val
            355                 360                 365
tct ttc gga gtg tgg att cgc act cct cca gct tat aga cca cca aat       1152
Ser Phe Gly Val Trp Ile Arg Thr Pro Pro Ala Tyr Arg Pro Pro Asn
        370                 375                 380
gcc cct atc tta tca aca ctt ccg gag act act gtt gtt taa gctt          1198
Ala Pro Ile Leu Ser Thr Leu Pro Glu Thr Thr Val Val
    385                 390                 395
```

<210> 5
<211> 396
<212> PRT
<213> Artificial sequence
<220>
<223> CWekGlyKatwPIG
<400> 5

```
Met Asp Ile Asp Pro Tyr Lys Glu Phe Gly Ala Thr Val Glu Leu Leu
1               5                   10                  15
Ser Phe Leu Pro Ser Asp Phe Phe Pro Ser Val Arg Asp Leu Leu Asp
            20                  25                  30
Thr Ala Ser Ala Leu Tyr Arg Glu Ala Leu Glu Ser Pro Glu His Cys
        35                  40                  45
Ser Pro His His Thr Ala Leu Arg Gln Ala Ile Leu Cys Trp Gly Glu
    50                  55                  60
Leu Met Thr Leu Ala Thr Trp Val Gly Val Asn Leu Glu Asp Gln Gly
65                  70                  75                  80
Gly Gly Gly Ser Gly Gly Gly Gly Gln Gly Gly Ala Val Pro Asp Lys
                85                  90                  95
Ile Asp Trp Arg Glu Ser Gly Tyr Val Thr Glu Val Lys Asp Gln Gly
            100                 105                 110
Asn Cys Gly Ser Cys Trp Ala Phe Ser Thr Thr Gly Thr Met Glu Gly
        115                 120                 125
Gln Tyr Met Lys Asn Glu Arg Thr Ser Ile Ser Phe Ser Glu Gln Gln
    130                 135                 140
Leu Val Asp Cys Ser Gly Pro Trp Gly Asn Asn Gly Cys Ser Gly Gly
145                 150                 155                 160
Leu Met Glu Asn Ala Tyr Gln Tyr Leu Lys Gln Phe Gly Leu Glu Thr
                165                 170                 175
Glu Ser Ser Tyr Pro Tyr Thr Ala Val Glu Gly Gln Cys Arg Tyr Asn
            180                 185                 190

Lys Gln Leu Gly Val Ala Lys Val Thr Gly Tyr Tyr Thr Val Pro Ser
        195                 200                 205
Gly Ser Glu Val Glu Leu Lys Asn Leu Val Gly Ala Glu Gly Pro Ala
    210                 215                 220
Ala Val Ala Val Asp Val Glu Ser Asp Phe Met Met Tyr Arg Ser Gly
225                 230                 235                 240
Ile Tyr Gln Ser Gln Thr Cys Ser Pro Leu Arg Val Asn His Ala Val
                245                 250                 255
Leu Ala Val Gly Tyr Gly Thr Gln Gly Gly Thr Asp Tyr Trp Ile Val
            260                 265                 270
Lys Asn Ser Trp Gly Leu Ser Trp Gly Glu Arg Gly Tyr Ile Arg Met
        275                 280                 285
Ala Arg Asn Arg Gly Asn Met Cys Gly Ile Ala Ser Leu Ala Ser Leu
    290                 295                 300
Pro Ile Gly Thr Ile Ser Gly Ile Asp Gly Gln Gly Gly Gln Gly Gly
305                 310                 315                 320
Gly Gly Ser Gly Gly Gly Gly Ser Arg Asp Leu Val Val Ser Tyr Val
                325                 330                 335
Asn Thr Asn Met Gly Leu Lys Phe Arg Gln Leu Leu Trp Phe His Ile
            340                 345                 350
Ser Cys Leu Thr Phe Gly Arg Glu Thr Val Ile Glu Tyr Leu Val Ser
        355                 360                 365
Phe Gly Val Trp Ile Arg Thr Pro Pro Ala Tyr Arg Pro Pro Asn Ala
    370                 375                 380
Pro Ile Leu Ser Thr Leu Pro Glu Thr Thr Val Val
385                 390                 395
```

<210> 6
<211> 1127
<212> DNA
<213> artificial sequence
<220>
<223> Moty_cat_Ckrd

<220>
<221> CDS
<222> (9)..(1115)
<223> sequence coding the hybrid protein HBV core domain::catalytic dom ain of the liver fluke cysteine protease
<400> 6

```
ggatccat atg gct gta ccc gac aaa att gac tgg cgt gaa tct ggt tat      50
         Met Ala Val Pro Asp Lys Ile Asp Trp Arg Glu Ser Gly Tyr
         1               5                   10
gtg acg gag gtg aaa gat cag gga aac tgt ggt tcc tgt tgg gca ttc      98
Val Thr Glu Val Lys Asp Gln Gly Asn Cys Gly Ser Cys Trp Ala Phe
15                  20                  25                  30
tca aca acc ggt act atg gag gga cag tat atg aaa aac gaa aga act     146
Ser Thr Thr Gly Thr Met Glu Gly Gln Tyr Met Lys Asn Glu Arg Thr
                35                  40                  45
agt att tca ttc tct gag caa caa ctg gtc gat tgt agc ggt cct tgg     194
Ser Ile Ser Phe Ser Glu Gln Gln Leu Val Asp Cys Ser Gly Pro Trp
            50                  55                  60
gga aat aat ggt tgc agt ggt gga ttg atg gaa aat gct tac caa tat     242
Gly Asn Asn Gly Cys Ser Gly Gly Leu Met Glu Asn Ala Tyr Gln Tyr
        65                  70                  75
ttg aaa caa ttt gga ttg gaa acc gaa tcc tct tat ccg tac acg gct     290
Leu Lys Gln Phe Gly Leu Glu Thr Glu Ser Ser Tyr Pro Tyr Thr Ala
    80                  85                  90
gtg gaa ggt cag tgt cga tac aat aag cag tta gga gtt gcc aaa gtg     338
Val Glu Gly Gln Cys Arg Tyr Asn Lys Gln Leu Gly Val Ala Lys Val
95                  100                 105                 110
act ggc tac tac act gtg cct tct ggc agt gag gta gaa ttg aaa aat     386
Thr Gly Tyr Tyr Thr Val Pro Ser Gly Ser Glu Val Glu Leu Lys Asn
```

```
                115                 120                 125
cta gtc ggt gcc gaa gga cct gcc gcg gtc gct gtg gat gtg gaa tct      434
Leu Val Gly Ala Glu Gly Pro Ala Ala Val Ala Val Asp Val Glu Ser
            130                 135                 140
gac ttc atg atg tac agg agt ggt att tat cag agc caa act tgt tca      482
Asp Phe Met Met Tyr Arg Ser Gly Ile Tyr Gln Ser Gln Thr Cys Ser
        145                 150                 155
ccg ctt cgt gtg aat cat gca gtc ttg gct gtc ggt tac gga aca cag      530
Pro Leu Arg Val Asn His Ala Val Leu Ala Val Gly Tyr Gly Thr Gln
    160                 165                 170
ggt ggt act gac tat tgg att gtg aaa aat agt tgg gga ttg tcg tgg      578
Gly Gly Thr Asp Tyr Trp Ile Val Lys Asn Ser Trp Gly Leu Ser Trp
175                 180                 185                 190
ggt gag cgc ggt tac att cga atg gct agg aat cga ggt aac atg tgt      626
Gly Glu Arg Gly Tyr Ile Arg Met Ala Arg Asn Arg Gly Asn Met Cys
                195                 200                 205
gga att gct tcg ctg gcc agt ctc ccg att ggc acg att tcc ggc atc      674
Gly Ile Ala Ser Leu Ala Ser Leu Pro Ile Gly Thr Ile Ser Gly Ile
            210                 215                 220
gat cct tat aaa gaa ttt gga gct act gtg gag tta ctc tcg ttt ttg      722
Asp Pro Tyr Lys Glu Phe Gly Ala Thr Val Glu Leu Leu Ser Phe Leu
        225                 230                 235
cct tct gac ttc ttt cct tca gta cga gat ctt cta gat acc gcc tca      770
Pro Ser Asp Phe Phe Pro Ser Val Arg Asp Leu Leu Asp Thr Ala Ser
    240                 245                 250
gct ctc tat cgg gaa gcc tta gag tct cct gag cat tgt tca cct cac      818
Ala Leu Tyr Arg Glu Ala Leu Glu Ser Pro Glu His Cys Ser Pro His
255                 260                 265                 270
cat act gca ctc agg caa gca att ctt tgc tgg ggg gaa cta atg act      866
His Thr Ala Leu Arg Gln Ala Ile Leu Cys Trp Gly Glu Leu Met Thr
                275                 280                 285
cta gct acc tgg gtg ggt gtt aat ttg gaa gat cca gca tct agg gac      914
Leu Ala Thr Trp Val Gly Val Asn Leu Glu Asp Pro Ala Ser Arg Asp
            290                 295                 300
cta gtt gtt agt tat gtc aac act aat atg ggc tta aag ttc agg caa      962
Leu Val Val Ser Tyr Val Asn Thr Asn Met Gly Leu Lys Phe Arg Gln
        305                 310                 315
ctt ttg tgg ttt cac att tct tgt ctc act ttt gga aga gaa acg gtc     1010
Leu Leu Trp Phe His Ile Ser Cys Leu Thr Phe Gly Arg Glu Thr Val
    320                 325                 330
ata gag tat ttg gtg tct ttc gga gtg tgg att cgc act cct cca gct     1058
Ile Glu Tyr Leu Val Ser Phe Gly Val Trp Ile Arg Thr Pro Pro Ala
335                 340                 345                 350
tat aga cca cca aat gcc cct atc tta tca aca ctt ccg gag act act     1106
Tyr Arg Pro Pro Asn Ala Pro Ile Leu Ser Thr Leu Pro Glu Thr Thr
                355                 360                 365
gtt gtt taa aagcttgagc tc                                          1127
Val Val
```

<210> 7
<211> 368
<212> PRT
<213> artificial sequence
<220>
<223> Moty_cat_Ckrd
<400> 7

```
Met Ala Val Pro Asp Lys Ile Asp Trp Arg Glu Ser Gly Tyr Val Thr
1               5                   10                  15
Glu Val Lys Asp Gln Gly Asn Cys Gly Ser Cys Trp Ala Phe Ser Thr
            20                  25                  30
Thr Gly Thr Met Glu Gly Gln Tyr Met Lys Asn Glu Arg Thr Ser Ile
        35                  40                  45
Ser Phe Ser Glu Gln Gln Leu Val Asp Cys Ser Gly Pro Trp Gly Asn
    50                  55                  60
Asn Gly Cys Ser Gly Gly Leu Met Glu Asn Ala Tyr Gln Tyr Leu Lys
65                  70                  75                  80
Gln Phe Gly Leu Glu Thr Glu Ser Ser Tyr Pro Tyr Thr Ala Val Glu
                85                  90                  95
Gly Gln Cys Arg Tyr Asn Lys Gln Leu Gly Val Ala Lys Val Thr Gly
            100                 105                 110
Tyr Tyr Thr Val Pro Ser Gly Ser Glu Val Glu Leu Lys Asn Leu Val
        115                 120                 125
Gly Ala Glu Gly Pro Ala Ala Val Ala Val Asp Val Glu Ser Asp Phe
    130                 135                 140
Met Met Tyr Arg Ser Gly Ile Tyr Gln Ser Gln Thr Cys Ser Pro Leu
145                 150                 155                 160
Arg Val Asn His Ala Val Leu Ala Val Gly Tyr Gly Thr Gln Gly Gly
                165                 170                 175
Thr Asp Tyr Trp Ile Val Lys Asn Ser Trp Gly Leu Ser Trp Gly Glu
            180                 185                 190
Arg Gly Tyr Ile Arg Met Ala Arg Asn Arg Gly Asn Met Cys Gly Ile
        195                 200                 205
Ala Ser Leu Ala Ser Leu Pro Ile Gly Thr Ile Ser Gly Ile Asp Pro
    210                 215                 220
Tyr Lys Glu Phe Gly Ala Thr Val Glu Leu Leu Ser Phe Leu Pro Ser
225                 230                 235                 240
Asp Phe Phe Pro Ser Val Arg Asp Leu Leu Asp Thr Ala Ser Ala Leu
                245                 250                 255
Tyr Arg Glu Ala Leu Glu Ser Pro Glu His Cys Ser Pro His His Thr
            260                 265                 270
Ala Leu Arg Gln Ala Ile Leu Cys Trp Gly Glu Leu Met Thr Leu Ala
        275                 280                 285
Thr Trp Val Gly Val Asn Leu Glu Asp Pro Ala Ser Arg Asp Leu Val
    290                 295                 300
Val Ser Tyr Val Asn Thr Asn Met Gly Leu Lys Phe Arg Gln Leu Leu
305                 310                 315                 320
Trp Phe His Ile Ser Cys Leu Thr Phe Gly Arg Glu Thr Val Ile Glu
                325                 330                 335
Tyr Leu Val Ser Phe Gly Val Trp Ile Arg Thr Pro Pro Ala Tyr Arg
            340                 345                 350
Pro Pro Asn Ala Pro Ile Leu Ser Thr Leu Pro Glu Thr Thr Val Val
        355                 360                 365
```

<210> 8
<211> 699
<212> DNA
<213> artificial sequence
<220>
<223> KAT_Nasza_MOT1_MOT2
<220>
<221> CDS
<222> (9)..(695)

<223> sequence coding the catalytic domain of the liver fluke cysteine protease

<400> 8

```
ggatccat atg gct gta ccc gac aaa att gac tgg cgt gaa tct ggt tat      50
         Met Ala Val Pro Asp Lys Ile Asp Trp Arg Glu Ser Gly Tyr
         1               5                   10
gtg acg gag gtg aaa gat cag gga aac tgt ggt tcc tgt tgg gca ttc       98
Val Thr Glu Val Lys Asp Gln Gly Asn Cys Gly Ser Cys Trp Ala Phe
15                  20                  25                  30
tca aca acc ggt act atg gag gga cag tat atg aaa aac gaa aga act      146
Ser Thr Thr Gly Thr Met Glu Gly Gln Tyr Met Lys Asn Glu Arg Thr

                35                  40                  45
agt att tca ttc tct gag caa caa ctg gtc gat tgt agc ggt cct tgg      194
Ser Ile Ser Phe Ser Glu Gln Gln Leu Val Asp Cys Ser Gly Pro Trp
            50                  55                  60
gga aat aat ggt tgc agt ggt gga ttg atg gaa aat gct tac caa tat      242
Gly Asn Asn Gly Cys Ser Gly Gly Leu Met Glu Asn Ala Tyr Gln Tyr
        65                  70                  75
ttg aaa caa ttt gga ttg gaa acc gaa tcc tct tat ccg tac acg gct      290
Leu Lys Gln Phe Gly Leu Glu Thr Glu Ser Ser Tyr Pro Tyr Thr Ala
    80                  85                  90
gtg gaa ggt cag tgt cga tac aat aag cag tta gga gtt gcc aaa gtg      338
Val Glu Gly Gln Cys Arg Tyr Asn Lys Gln Leu Gly Val Ala Lys Val
95                  100                 105                 110
act ggc tac tac act gtg cct tct ggc agt gag gta gaa ttg aaa aat      386
Thr Gly Tyr Tyr Thr Val Pro Ser Gly Ser Glu Val Glu Leu Lys Asn
                115                 120                 125
cta gtc ggt gcc gaa gga cct gcc gcg gtc gct gtg gat gtg gaa tct      434
Leu Val Gly Ala Glu Gly Pro Ala Ala Val Ala Val Asp Val Glu Ser
            130                 135                 140
gac ttc atg atg tac agg agt ggt att tat cag agc caa act tgt tca      482
Asp Phe Met Met Tyr Arg Ser Gly Ile Tyr Gln Ser Gln Thr Cys Ser
        145                 150                 155
ccg ctt cgt gtg aat cat gca gtc ttg gct gtc ggt tac gga aca cag      530
Pro Leu Arg Val Asn His Ala Val Leu Ala Val Gly Tyr Gly Thr Gln
    160                 165                 170
ggt ggt act gac tat tgg att gtg aaa aat agt tgg gga ttg tcg tgg      578
Gly Gly Thr Asp Tyr Trp Ile Val Lys Asn Ser Trp Gly Leu Ser Trp
175                 180                 185                 190
ggt gag cgc ggt tac att cga atg gct agg aat cga ggt aac atg tgt      626
Gly Glu Arg Gly Tyr Ile Arg Met Ala Arg Asn Arg Gly Asn Met Cys
                195                 200                 205
gga att gct tcg ctg gcc agt ctc ccg atg gtg gca cga ttt ccg ggt      674
Gly Ile Ala Ser Leu Ala Ser Leu Pro Met Val Ala Arg Phe Pro Gly
            210                 215                 220
agc ggt agt gat atc gat taa gctt                                     699
Ser Gly Ser Asp Ile Asp
        225
```

<210> 9

<211> 228

<212> PRT

<213> artificial sequence

<220>

<223> KAT_Nasza_MOT1_MOT2

<400> 9

```
Met Ala Val Pro Asp Lys Ile Asp Trp Arg Glu Ser Gly Tyr Val Thr
1               5                   10                  15
Glu Val Lys Asp Gln Gly Asn Cys Gly Ser Cys Trp Ala Phe Ser Thr
            20                  25                  30
Thr Gly Thr Met Glu Gly Gln Tyr Met Lys Asn Glu Arg Thr Ser Ile
        35                  40                  45
Ser Phe Ser Glu Gln Gln Leu Val Asp Cys Ser Gly Pro Trp Gly Asn
    50                  55                  60
Asn Gly Cys Ser Gly Gly Leu Met Glu Asn Ala Tyr Gln Tyr Leu Lys
65                  70                  75                  80
Gln Phe Gly Leu Glu Thr Glu Ser Ser Tyr Pro Tyr Thr Ala Val Glu
                85                  90                  95
Gly Gln Cys Arg Tyr Asn Lys Gln Leu Gly Val Ala Lys Val Thr Gly
            100                 105                 110
Tyr Tyr Thr Val Pro Ser Gly Ser Glu Val Glu Leu Lys Asn Leu Val
        115                 120                 125
Gly Ala Glu Gly Pro Ala Ala Val Ala Val Asp Val Glu Ser Asp Phe
    130                 135                 140
Met Met Tyr Arg Ser Gly Ile Tyr Gln Ser Gln Thr Cys Ser Pro Leu
145                 150                 155                 160
Arg Val Asn His Ala Val Leu Ala Val Gly Tyr Gly Thr Gln Gly Gly
                165                 170                 175
Thr Asp Tyr Trp Ile Val Lys Asn Ser Trp Gly Leu Ser Trp Gly Glu
            180                 185                 190
Arg Gly Tyr Ile Arg Met Ala Arg Asn Arg Gly Asn Met Cys Gly Ile
        195                 200                 205
Ala Ser Leu Ala Ser Leu Pro Met Val Ala Arg Phe Pro Gly Ser Gly
    210                 215                 220
Ser Asp Ile Asp
225
```

<210> 10
<211> 694
<212> DNA
<213> artificial sequence
<220>
<223> sequence coding the catalytic domain of the liver fluke cysteine protease
<220>
<221> CDS
<222> (4)..(690)
<223> sequence coding the catalytic domain of the liver fluke cysteine protease (fig.11)
<400> 10

```
cat atg gct gta ccc gac aaa att gac tgg cgt gaa tct ggt tat gtg       48
    Met Ala Val Pro Asp Lys Ile Asp Trp Arg Glu Ser Gly Tyr Val
    1               5                   10                  15
acg gag gtg aaa gat cag gga aac tgt ggt tcc tgt tgg gca ttc tca       96
Thr Glu Val Lys Asp Gln Gly Asn Cys Gly Ser Cys Trp Ala Phe Ser
                20                  25                  30
aca acc ggt act atg gag gga cag tat atg aaa aac gaa aga act agt      144
Thr Thr Gly Thr Met Glu Gly Gln Tyr Met Lys Asn Glu Arg Thr Ser
            35                  40                  45
att tca ttc tct gag caa caa ctg gtc gat tgt agc ggt cct tgg gga      192
Ile Ser Phe Ser Glu Gln Gln Leu Val Asp Cys Ser Gly Pro Trp Gly
        50                  55                  60
aat aat ggt tgc agt ggt gga ttg atg gaa aat gct tac caa tat ttg      240
Asn Asn Gly Cys Ser Gly Gly Leu Met Glu Asn Ala Tyr Gln Tyr Leu
    65                  70                  75
aaa caa ttt gga ttg gaa acc gaa tcc tct tat ccg tac acg gct gtg      288
Lys Gln Phe Gly Leu Glu Thr Glu Ser Ser Tyr Pro Tyr Thr Ala Val
80                  85                  90                  95
gaa ggt cag tgt cga tac aat aag cag tta gga gtt gcc aaa gtg act      336
Glu Gly Gln Cys Arg Tyr Asn Lys Gln Leu Gly Val Ala Lys Val Thr
                100                 105                 110
ggc tac tac act gtg cct tct ggc agt gag gta gaa ttg aaa aat cta      384
Gly Tyr Tyr Thr Val Pro Ser Gly Ser Glu Val Glu Leu Lys Asn Leu
            115                 120                 125
gtc ggt gcc gaa gga cct gcc gcg gtc gct gtg gat gtg gaa tct gac      432
Val Gly Ala Glu Gly Pro Ala Ala Val Ala Val Asp Val Glu Ser Asp
        130                 135                 140
ttc atg atg tac agg agt ggt att tat cag agc caa act tgt tca ccg      480
Phe Met Met Tyr Arg Ser Gly Ile Tyr Gln Ser Gln Thr Cys Ser Pro
    145                 150                 155
ctt cgt gtg aat cat gca gtc ttg gct gtc ggt tac gga aca cag ggt      528
Leu Arg Val Asn His Ala Val Leu Ala Val Gly Tyr Gly Thr Gln Gly
160                 165                 170                 175
ggt act gac tat tgg att gtg aaa aat agt tgg gga ttg tcg tgg ggt      576
Gly Thr Asp Tyr Trp Ile Val Lys Asn Ser Trp Gly Leu Ser Trp Gly
                180                 185                 190
gag cgc ggt tac att cga atg gct agg aat cga ggt aac atg tgt gga      624
Glu Arg Gly Tyr Ile Arg Met Ala Arg Asn Arg Gly Asn Met Cys Gly
            195                 200                 205
att gct tcg ctg gcc agt ctc ccg atg gtg gca cga ttt ccg ggt agc      672
Ile Ala Ser Leu Ala Ser Leu Pro Met Val Ala Arg Phe Pro Gly Ser
        210                 215                 220
ggt agt gat atc gat taa gctt                                         694
Gly Ser Asp Ile Asp
    225
```

<210> 11
<211> 228
<212> PRT
<213> artificial sequence
<220>
<223> sequence coding the catalytic domain of the liver fluke cysteine protease
<400> 11

```
Met Ala Val Pro Asp Lys Ile Asp Trp Arg Glu Ser Gly Tyr Val Thr
1               5                   10                  15
Glu Val Lys Asp Gln Gly Asn Cys Gly Ser Cys Trp Ala Phe Ser Thr
            20                  25                  30
Thr Gly Thr Met Glu Gly Gln Tyr Met Lys Asn Glu Arg Thr Ser Ile
        35                  40                  45
Ser Phe Ser Glu Gln Gln Leu Val Asp Cys Ser Gly Pro Trp Gly Asn
    50                  55                  60
Asn Gly Cys Ser Gly Gly Leu Met Glu Asn Ala Tyr Gln Tyr Leu Lys
65                  70                  75                  80
Gln Phe Gly Leu Glu Thr Glu Ser Ser Tyr Pro Tyr Thr Ala Val Glu
                85                  90                  95
Gly Gln Cys Arg Tyr Asn Lys Gln Leu Gly Val Ala Lys Val Thr Gly
            100                 105                 110
Tyr Tyr Thr Val Pro Ser Gly Ser Glu Val Glu Leu Lys Asn Leu Val
        115                 120                 125
Gly Ala Glu Gly Pro Ala Ala Val Ala Val Asp Val Glu Ser Asp Phe
    130                 135                 140
Met Met Tyr Arg Ser Gly Ile Tyr Gln Ser Gln Thr Cys Ser Pro Leu
145                 150                 155                 160
Arg Val Asn His Ala Val Leu Ala Val Gly Tyr Gly Thr Gln Gly Gly
                165                 170                 175
Thr Asp Tyr Trp Ile Val Lys Asn Ser Trp Gly Leu Ser Trp Gly Glu
            180                 185                 190
Arg Gly Tyr Ile Arg Met Ala Arg Asn Arg Gly Asn Met Cys Gly Ile
        195                 200                 205
Ala Ser Leu Ala Ser Leu Pro Met Val Ala Arg Phe Pro Gly Ser Gly
    210                 215                 220
Ser Asp Ile Asp
225
```

<210> 12
<211> 686
<212> DNA
<213> artificial sequence
<220>
<223> MOT_DO_C_POP_MOT1_MOT3_W_BLUESCRIPCIE
<220>
<221> gene
<222> (1)..(686)
<223>
<400> 12

```
ggatccatat ggctgtaccc gacaaaattg actggcgtga atctggttat gtgacggagg     60
tgaaagatca gggaaactgt ggttcctgtt gggcattctc aacaaccggt actatggagg    120
gacagtatat gaaaaacgaa agaactagta tttcattctc tgagcaacaa ctggtcgatt    180
gtagtggtcc ttggggaaat aatggttgca gtggtggatt gatggaaaat gcttaccaat    240
atttgaaaca atttggattg gaaaccgaat cctcttatcc gtacacggct gtggaaggtc    300
agtgtcgata caataagcag ttaggagttg ccaaagtgac tggctactac actgtgcctt    360
ctggcagtga ggtagaattg aaaaatctag tcggtgccga aggacctgcc gcggtcgctg    420
tggatgtgga atctgacttc atgatgtaca ggagtggtat ttatcagagc caaacttgtt    480
caccgcttcg tgtgaatcat gcagtcttgg ctgtcggtta cggaacacag ggtggtactg    540
actattggat tgtgaaaaat agttggggat tgtcgtgggg tgagcgcggt tacattcgaa    600
tggctaggaa tcgaggtaac atgtgtggaa ttgcttcgct ggccagtctc ccgatggtgg    660
cacgatttcc ggcatcgatt aagctt                                          686
```

<210> 13
<211> 681
<212> DNA
<213> artificial sequence
<220>
<223> MOT_DO_C_POP_MOT1_MOT3_W_PIG
<220>
<221> gene
<222> (1)..(681)
<223>
<400> 13

```
catatggctg tacccgacaa aattgactgg cgtgaatctg gttatgtgac ggaggtgaaa     60
gatcagggaa actgtggttc ctgttgggca ttctcaacaa ccggtactat ggagggacag    120
tatatgaaaa acgaaagaac tagtatttca ttctctgagc aacaactggt cgattgtagt    180
ggtccttggg gaaataatgg ttgcagtggt ggattgatgg aaaatgctta ccaatatttg    240
aaacaatttg gattggaaac cgaatcctct tatccgtaca cggctgtgga aggtcagtgt    300
cgatacaata agcagttagg agttgccaaa gtgactggct actacactgt gccttctggc    360
agtgaggtag aattgaaaaa tctagtcggt gccgaaggac ctgccgcggt cgctgtggat    420
gtggaatctg acttcatgat gtacaggagt ggtatttatc agagccaaac ttgttcaccg    480
cttcgtgtga atcatgcagt cttggctgtc ggttacggaa cacagggtgg tactgactat    540
tggattgtga aaaatagttg ggattgtcg tggggtgagc gcggttacat tcgaatggct    600
aggaatcgag gtaacatgtg tggaattgct tcgctggcca gtctcccgat ggtggcacga    660
tttccggcat cgattaagct t                                              681
```

<210> 14
<211> 1116
<212> DNA
<213> artificial sequence
<220>
<223> Moty_cat_Ckrd_w_PIG
<220>
<221> CDS
<222> (4)..(1110)
<223> sequence coding the hybrid protein HBV core domain::catalytic dom ain of the liver fluke cysteine protease
<400> 14

```
cat atg gct gta ccc gac aaa att gac tgg cgt gaa tct ggt tat gtg       48
    Met Ala Val Pro Asp Lys Ile Asp Trp Arg Glu Ser Gly Tyr Val
    1               5                   10                  15
acg gag gtg aaa gat cag gga aac tgt ggt tcc tgt tgg gca ttc tca       96
Thr Glu Val Lys Asp Gln Gly Asn Cys Gly Ser Cys Trp Ala Phe Ser
                20                  25                  30
aca acc ggt act atg gag gga cag tat atg aaa aac gaa aga act agt      144
Thr Thr Gly Thr Met Glu Gly Gln Tyr Met Lys Asn Glu Arg Thr Ser
            35                  40                  45
att tca ttc tct gag caa caa ctg gtc gat tgt agc ggt cct tgg gga      192
Ile Ser Phe Ser Glu Gln Gln Leu Val Asp Cys Ser Gly Pro Trp Gly
        50                  55                  60
aat aat ggt tgc agt ggt gga ttg atg gaa aat gct tac caa tat ttg      240
Asn Asn Gly Cys Ser Gly Gly Leu Met Glu Asn Ala Tyr Gln Tyr Leu
    65                  70                  75
aaa caa ttt gga ttg gaa acc gaa tcc tct tat ccg tac acg gct gtg      288
```

```
Lys Gln Phe Gly Leu Glu Thr Glu Ser Ser Tyr Pro Tyr Thr Ala Val
80                  85                  90                  95
gaa ggt cag tgt cga tac aat aag cag tta gga gtt gcc aaa gtg act        336
Glu Gly Gln Cys Arg Tyr Asn Lys Gln Leu Gly Val Ala Lys Val Thr
                100                 105                 110
ggc tac tac act gtg cct tct ggc agt gag gta gaa ttg aaa aat cta        384
Gly Tyr Tyr Thr Val Pro Ser Gly Ser Glu Val Glu Leu Lys Asn Leu
            115                 120                 125
gtc ggt gcc gaa gga cct gcc gcg gtc gct gtg gat gtg gaa tct gac        432
Val Gly Ala Glu Gly Pro Ala Ala Val Ala Val Asp Val Glu Ser Asp
        130                 135                 140
ttc atg atg tac agg agt ggt att tat cag agc caa act tgt tca ccg        480
Phe Met Met Tyr Arg Ser Gly Ile Tyr Gln Ser Gln Thr Cys Ser Pro
    145                 150                 155
ctt cgt gtg aat cat gca gtc ttg gct gtc ggt tac gga aca cag ggt        528
Leu Arg Val Asn His Ala Val Leu Ala Val Gly Tyr Gly Thr Gln Gly
160                 165                 170                 175
ggt act gac tat tgg att gtg aaa aat agt tgg gga ttg tcg tgg ggt        576
Gly Thr Asp Tyr Trp Ile Val Lys Asn Ser Trp Gly Leu Ser Trp Gly
                180                 185                 190
gag cgc ggt tac att cga atg gct agg aat cga ggt aac atg tgt gga        624
Glu Arg Gly Tyr Ile Arg Met Ala Arg Asn Arg Gly Asn Met Cys Gly
            195                 200                 205
att gct tcg ctg gcc agt ctc ccg att ggc acg att tcc ggc atc gat        672
Ile Ala Ser Leu Ala Ser Leu Pro Ile Gly Thr Ile Ser Gly Ile Asp
        210                 215                 220
cct tat aaa gaa ttt gga gct act gtg gag tta ctc tcg ttt ttg cct        720
Pro Tyr Lys Glu Phe Gly Ala Thr Val Glu Leu Leu Ser Phe Leu Pro
    225                 230                 235
tct gac ttc ttt cct tca gta cga gat ctt cta gat acc gcc tca gct        768
Ser Asp Phe Phe Pro Ser Val Arg Asp Leu Leu Asp Thr Ala Ser Ala
240                 245                 250                 255
ctc tat cgg gaa gcc tta gag tct cct gag cat tgt tca cct cac cat        816
Leu Tyr Arg Glu Ala Leu Glu Ser Pro Glu His Cys Ser Pro His His
                260                 265                 270
act gca ctc agg caa gca att ctt tgc tgg ggg gaa cta atg act cta        864
Thr Ala Leu Arg Gln Ala Ile Leu Cys Trp Gly Glu Leu Met Thr Leu
            275                 280                 285
gct acc tgg gtg ggt gtt aat ttg gaa gat cca gca tct agg gac cta        912
Ala Thr Trp Val Gly Val Asn Leu Glu Asp Pro Ala Ser Arg Asp Leu
        290                 295                 300
gtt gtt agt tat gtc aac act aat atg ggc tta aag ttc agg caa ctt        960
Val Val Ser Tyr Val Asn Thr Asn Met Gly Leu Lys Phe Arg Gln Leu
    305                 310                 315
ttg tgg ttt cac att tct tgt ctc act ttt gga aga gaa acg gtc ata       1008
Leu Trp Phe His Ile Ser Cys Leu Thr Phe Gly Arg Glu Thr Val Ile
320                 325                 330                 335
gag tat ttg gtg tct ttc gga gtg tgg att cgc act cct cca gct tat       1056
Glu Tyr Leu Val Ser Phe Gly Val Trp Ile Arg Thr Pro Pro Ala Tyr
                340                 345                 350
aga cca cca aat gcc cct atc tta tca aca ctt ccg gag act act gtt       1104
Arg Pro Pro Asn Ala Pro Ile Leu Ser Thr Leu Pro Glu Thr Thr Val
            355                 360                 365
gtt taa aagctt                                                        1116
Val
```

<210> 15
<211> 368
<212> PRT

<213> artificial sequence
<220>
<223> Moty_cat_Ckrd_w_PIG
<400> 15

```
Met Ala Val Pro Asp Lys Ile Asp Trp Arg Glu Ser Gly Tyr Val Thr
1               5                   10                  15
Glu Val Lys Asp Gln Gly Asn Cys Gly Ser Cys Trp Ala Phe Ser Thr
            20                  25                  30
Thr Gly Thr Met Glu Gly Gln Tyr Met Lys Asn Glu Arg Thr Ser Ile
        35                  40                  45
Ser Phe Ser Glu Gln Gln Leu Val Asp Cys Ser Gly Pro Trp Gly Asn
    50                  55                  60
Asn Gly Cys Ser Gly Gly Leu Met Glu Asn Ala Tyr Gln Tyr Leu Lys
65                  70                  75                  80
Gln Phe Gly Leu Glu Thr Glu Ser Ser Tyr Pro Tyr Thr Ala Val Glu
                85                  90                  95
Gly Gln Cys Arg Tyr Asn Lys Gln Leu Gly Val Ala Lys Val Thr Gly
            100                 105                 110
Tyr Tyr Thr Val Pro Ser Gly Ser Glu Val Glu Leu Lys Asn Leu Val
        115                 120                 125
Gly Ala Glu Gly Pro Ala Ala Val Ala Val Asp Val Glu Ser Asp Phe
    130                 135                 140
Met Met Tyr Arg Ser Gly Ile Tyr Gln Ser Gln Thr Cys Ser Pro Leu
145                 150                 155                 160
Arg Val Asn His Ala Val Leu Ala Val Gly Tyr Gly Thr Gln Gly Gly
                165                 170                 175
Thr Asp Tyr Trp Ile Val Lys Asn Ser Trp Gly Leu Ser Trp Gly Glu
            180                 185                 190
Arg Gly Tyr Ile Arg Met Ala Arg Asn Arg Gly Asn Met Cys Gly Ile
        195                 200                 205
Ala Ser Leu Ala Ser Leu Pro Ile Gly Thr Ile Ser Gly Ile Asp Pro
    210                 215                 220
Tyr Lys Glu Phe Gly Ala Thr Val Glu Leu Leu Ser Phe Leu Pro Ser
225                 230                 235                 240
Asp Phe Phe Pro Ser Val Arg Asp Leu Leu Asp Thr Ala Ser Ala Leu
                245                 250                 255
Tyr Arg Glu Ala Leu Glu Ser Pro Glu His Cys Ser Pro His His Thr
            260                 265                 270
Ala Leu Arg Gln Ala Ile Leu Cys Trp Gly Glu Leu Met Thr Leu Ala
        275                 280                 285
Thr Trp Val Gly Val Asn Leu Glu Asp Pro Ala Ser Arg Asp Leu Val
    290                 295                 300
Val Ser Tyr Val Asn Thr Asn Met Gly Leu Lys Phe Arg Gln Leu Leu
305                 310                 315                 320
Trp Phe His Ile Ser Cys Leu Thr Phe Gly Arg Glu Thr Val Ile Glu
                325                 330                 335
Tyr Leu Val Ser Phe Gly Val Trp Ile Arg Thr Pro Pro Ala Tyr Arg
            340                 345                 350
Pro Pro Asn Ala Pro Ile Leu Ser Thr Leu Pro Glu Thr Thr Val Val
        355                 360                 365
```

<210> 16
<211> 952
<212> DNA
<213> artificial sequence
<220>
<223> Cala_Mot_N
<220>
<221> CDS

<222> (4)..(948)
<223> sequence coding the precursor protein of the liver fluke cysteine protease (both the catalytic and leading domain)
<400> 16

```
cat atg tcg aat gat gat ttg tgg cat cag tgg aag cga atg tac aac      48
    Met Ser Asn Asp Asp Leu Trp His Gln Trp Lys Arg Met Tyr Asn
    1               5                   10                  15
aat gaa tac aat ggg gct gac gat cag cac aga cga aat att tgg gaa      96
Asn Glu Tyr Asn Gly Ala Asp Asp Gln His Arg Arg Asn Ile Trp Glu
                20                  25                  30
gag aat gtg aaa cat atc caa gaa cat aac cta cgt cac gat ctc ggc     144
Glu Asn Val Lys His Ile Gln Glu His Asn Leu Arg His Asp Leu Gly
            35                  40                  45
ctc gtc acc tac aca ttg gga ttg aac caa ttc acg gat atg aca ttc     192
Leu Val Thr Tyr Thr Leu Gly Leu Asn Gln Phe Thr Asp Met Thr Phe
        50                  55                  60
gag gaa ttc aag gcc aaa tat cta aca gaa atg tca cgc gcg tcc gat     240
Glu Glu Phe Lys Ala Lys Tyr Leu Thr Glu Met Ser Arg Ala Ser Asp
    65                  70                  75
ata ctc tca cac ggt gtc ccg tat gag acg aac aat cgt gcc gta ccc     288
Ile Leu Ser His Gly Val Pro Tyr Glu Thr Asn Asn Arg Ala Val Pro
80                  85                  90                  95
gac aaa att gac tgg cgt gaa tct ggt tat gtg acg gag gtg aaa gat     336
Asp Lys Ile Asp Trp Arg Glu Ser Gly Tyr Val Thr Glu Val Lys Asp
                100                 105                 110
cag gga aac tgt ggt tcc tgt tgg gca ttc tca aca acc ggt act atg     384
Gln Gly Asn Cys Gly Ser Cys Trp Ala Phe Ser Thr Thr Gly Thr Met
            115                 120                 125
gag gga cag tat atg aaa aac gaa aga act agt att tca ttc tct gag     432
Glu Gly Gln Tyr Met Lys Asn Glu Arg Thr Ser Ile Ser Phe Ser Glu
        130                 135                 140
caa caa ctg gtc gat tgt agc ggt cct tgg gga aat aat ggt tgc agt     480
Gln Gln Leu Val Asp Cys Ser Gly Pro Trp Gly Asn Asn Gly Cys Ser
    145                 150                 155
ggt gga ttg atg gaa aat gct tac caa tat ttg aaa caa ttt gga ttg     528
Gly Gly Leu Met Glu Asn Ala Tyr Gln Tyr Leu Lys Gln Phe Gly Leu
160                 165                 170                 175
gaa acc gaa tcc tct tat ccg tac acg gct gtg gaa ggt cag tgt cga     576
Glu Thr Glu Ser Ser Tyr Pro Tyr Thr Ala Val Glu Gly Gln Cys Arg
                180                 185                 190
tac aat aag cag tta gga gtt gcc aaa gtg act ggc tac tac act gtg     624
Tyr Asn Lys Gln Leu Gly Val Ala Lys Val Thr Gly Tyr Tyr Thr Val
            195                 200                 205
cct tct ggc agt gag gta gaa ttg aaa aat cta gtc ggt gcc gaa gga     672
Pro Ser Gly Ser Glu Val Glu Leu Lys Asn Leu Val Gly Ala Glu Gly
        210                 215                 220
cct gcc gcg gtc gct gtg gat gtg gaa tct gac ttc atg atg tac agg     720
Pro Ala Ala Val Ala Val Asp Val Glu Ser Asp Phe Met Met Tyr Arg
    225                 230                 235
agt ggt att tat cag agc caa act tgt tca ccg ctt cgt gtg aat cat     768
Ser Gly Ile Tyr Gln Ser Gln Thr Cys Ser Pro Leu Arg Val Asn His
240                 245                 250                 255
gca gtc ttg gct gtc ggt tac gga aca cag ggt ggt act gac tat tgg     816
Ala Val Leu Ala Val Gly Tyr Gly Thr Gln Gly Gly Thr Asp Tyr Trp
                260                 265                 270
att gtg aaa aat agt tgg gga ttg tcg tgg ggt gag cgc ggt tac att     864
Ile Val Lys Asn Ser Trp Gly Leu Ser Trp Gly Glu Arg Gly Tyr Ile
            275                 280                 285
cga atg gct agg aat cga ggt aac atg tgt gga att gct tcg ctg gcc     912
Arg Met Ala Arg Asn Arg Gly Asn Met Cys Gly Ile Ala Ser Leu Ala
        290                 295                 300
agt ctc ccg att ggc acg att tcc ggc atc gat taa gctt                952
Ser Leu Pro Ile Gly Thr Ile Ser Gly Ile Asp
    305                 310
```

<210> 17
<211> 314
<212> PRT
<213> artificial sequence
<220>
<223> Cala_Mot_N
<400> 17

```
Met Ser Asn Asp Asp Leu Trp His Gln Trp Lys Arg Met Tyr Asn Asn
1               5                   10                  15
Glu Tyr Asn Gly Ala Asp Asp Gln His Arg Arg Asn Ile Trp Glu Glu
            20                  25                  30
Asn Val Lys His Ile Gln Glu His Asn Leu Arg His Asp Leu Gly Leu
        35                  40                  45
Val Thr Tyr Thr Leu Gly Leu Asn Gln Phe Thr Asp Met Thr Phe Glu
    50                  55                  60
Glu Phe Lys Ala Lys Tyr Leu Thr Glu Met Ser Arg Ala Ser Asp Ile
65                  70                  75                  80
Leu Ser His Gly Val Pro Tyr Glu Thr Asn Asn Arg Ala Val Pro Asp
                85                  90                  95
Lys Ile Asp Trp Arg Glu Ser Gly Tyr Val Thr Glu Val Lys Asp Gln
            100                 105                 110
Gly Asn Cys Gly Ser Cys Trp Ala Phe Ser Thr Thr Gly Thr Met Glu
        115                 120                 125
Gly Gln Tyr Met Lys Asn Glu Arg Thr Ser Ile Ser Phe Ser Glu Gln
    130                 135                 140
Gln Leu Val Asp Cys Ser Gly Pro Trp Gly Asn Asn Gly Cys Ser Gly
145                 150                 155                 160
Gly Leu Met Glu Asn Ala Tyr Gln Tyr Leu Lys Gln Phe Gly Leu Glu
                165                 170                 175
Thr Glu Ser Ser Tyr Pro Tyr Thr Ala Val Glu Gly Gln Cys Arg Tyr
            180                 185                 190
Asn Lys Gln Leu Gly Val Ala Lys Val Thr Gly Tyr Tyr Thr Val Pro
        195                 200                 205
Ser Gly Ser Glu Val Glu Leu Lys Asn Leu Val Gly Ala Glu Gly Pro
    210                 215                 220
Ala Ala Val Ala Val Asp Val Glu Ser Asp Phe Met Met Tyr Arg Ser
225                 230                 235                 240
Gly Ile Tyr Gln Ser Gln Thr Cys Ser Pro Leu Arg Val Asn His Ala
                245                 250                 255
Val Leu Ala Val Gly Tyr Gly Thr Gln Gly Gly Thr Asp Tyr Trp Ile
            260                 265                 270
Val Lys Asn Ser Trp Gly Leu Ser Trp Gly Glu Arg Gly Tyr Ile Arg
        275                 280                 285
Met Ala Arg Asn Arg Gly Asn Met Cys Gly Ile Ala Ser Leu Ala Ser
    290                 295                 300
Leu Pro Ile Gly Thr Ile Ser Gly Ile Asp
305                 310
```

## Description

## FIELD OF THE INVENTION

The topic of the present invention is an oral vaccine and the methods for its production. In general, the present invention relates to the induction of an immune response, encompassing the administration of a vaccine through the gastrointestinal tract. The topics of the invention are used to obtain oral vaccines against the liver fluke.

## BACKGROUND OF THE INVENTION

The liver fluke (*Fasciola hepatica*) is a parasite which undergoes a complex life cycle, whose intermediate host is a snail, *Galba truncatula,* and whose definitive hosts are: domestic animals (cattle, sheep, goats and even horses), wild animals (wisent, elk, deer, squirrels - Vaughan et al., 1997; Aust. Vet. J. 75 (11): 811-3), and man. The definitive host become infected by ingesting metacercaria (one of the developmental forms of the liver fluke) found on plants and in the water. In the digestive tract, juvenile flukes undergo excystation, which penetrate the intestinal wall, the peritoneal cavity and the hepatic sac on their way to the hepatic tissues. After several weeks they reach the bile ducts, which is the habitat of the adult fluke. In addition to the bile ducts, invasions also occur in other tissues, i.e. the lungs. The disease caused by the liver fluke is described as fasciolosis. Liver fluke infections are a cause of hepatic damage, bile-duct damage, bile contamination, and bleeding. Liver fluke infections cause: anaemia, cirrhosis of the liver, and due to this a loss of productivity (significant weight loss, low milk production, low meat production, and low wool quality in sheep). More severe liver fluke infections can be fatal.

Pharmacological treatment is expensive, and increasingly ineffective due to a developing resistance to drugs. This parasite's invasion in Poland reaches from 10% of the sheep population (the north-eastern regions) to 98% (in the foothills). In Australia it is thought that 40 million sheep and 6 million cattle graze on land endemic to the liver fluke. Farmers spend some $10 million annually on fasciolosis treatment, and production losses total another $50-80 million (Boray J.C., 1999; NSW Agriculture Agfact (2nd ed.) A0.9.57) *Fasciola hepatica* is placed among the most significant veterinary problems, due to the dispersion of the parasite throughout the world, and the wide spectrum of host organisms.

It is thought that the best method for combating liver fluke infestations would be vaccinations. Their chief advantage is that they protect the host organism against damage in the pre-pathological period, at the same time ensuring immunity for many years. However, despite many attempts to construct vaccines against the liver fluke, none are available commercially.

Oral vaccines, in contrast to pareneteral ones, can ensure not only systemic immunity, but mucous membrane immunity as well. The latter is of particular importance in preventing gastro-intestinal parasite infections. The growing interest in oral vaccines is also fuelled by their ease of administration, which enables them to be used on a massive scale (Richter L., Kipp B.B., 1999, Curr. Top Microbiol. Immunol., 240, 159-176). First attempts have been made to produce oral vaccines. Description WO00/37610 describes vectors used to obtain immunogenic peptides, such as HBsAg, in edible plant tissues. Transgenic plants produced in this fashion are used to produce oral vaccines.

They are not without their disadvantages, however. It is difficult to ensurw a high and consistent enough dose of the vaccinating antigen. The level of the synthesised antigen varies in different tissues, and between individual transgenic plants.

Descriptions US 5770214, US 5811105 and US5804194 relate to the application of attenuated strains of *Salmonella typhi* as an oral vaccine.

Description US6290962, published 2001.09.18, describes a method of inducing an immune response against *Helicobacter* based on the administration of an immunogenic amount of antigen, which is a *Helicobacter* urease or a subunit thereof. Similar oral vaccines containing recombined proteins of *Helicobacter* urease produced in *E.coli* cells are the topic of Polish patents PL 179149 and PL 174448.

The relative inexperience in oral vaccination, and the unpredictable fate of the antigen in the gastro-intestinal tract make the conditions for this type of immunisation less well defined than for parenteral immunisation. Furthermore, oral immunisation carries the risk of inducing food-borne resistance (McGhee J.R.et al., 1999. in : Mucosal ImmunologyOgra P.L., Lamm M.E., Bienenstock J., Mestecky J., Strober W., McGhee J.R., red), str. 485-505, Academic Press) .

Description WO 98/32427 published 1998.07.30 describes a polymer used to prepare coated oral vaccines with delayed release of the active ingredient.

Regardless of the pathway of administration, the form of the antigen to a large degree warrants how immunogenic it is.

The acquisition of immunity as a result of vaccination requires adherence to an appropriate immunisation regime which encompasses the description of such parameters as the size and number of antigen doses, as well as the interval between immunisations. The acidic pH of gastric juices, proteolytic enzymes, and gut peristalsis force oral vaccine doses to be much higher than parenteral doses. At the same time, administering doses which are too high through the gastrointestinal tract can cause the above mentioned food-borne resistance. This does not mean, however, that low doses cannot cause this as well. To date, the basic mechanisms governing this phenomenon have not been discerned, and no standard method has been found for resolving the problem of induced resisitance. It is thought that the phenomenon of resistance is to some degree dependent on the above mentioned factors, meaning the size of the dose, immunisation schedule and type of antigen (Tacket C.O., Mason H.S., 1999, Microbes and Infection 1, 777-783). In the light of research to date, it seems that the perspectives of obtaining universal adjuvants, optimal vaccine compositions, efficacious doses and immunisation schedules for oral vaccines are very distant. (Makela P.H., 2000, FEMS Microbiology Reviews 24 , 9-20).

It seems that immunisation through the mucous membranes is conditional upon the multiple administration of an antigen in doses which are many times larger than effective doses for parenteral vaccination. Antigens forming multimers are more preferential. The effectiveness of adjuvant usage is also uncertain. In the case of edible plant vaccines the latter seems undesirable, but may be necessary for some antigens (Richter L., Kipp B.B, 1999, Curr. Top Microbiol. Immunol. 240, 159-176).

In summary, from the nature of oral vaccinations, it stems that the determination of a protocol of effective immunisation through the gastrointestinal tract is much more difficult than for vaccination using traditional methods. For each individual vaccine, this is a separate challenge burdened with a high risk of failure.

There are several indicators of the effectiveness of immunisation through the mucous membranes, including the gastrointestinal tract. These include the level of antigen-specific IgA class antibodies (S-IgA) connected with the mucous membranes, the serum titre of IgG and IgA class antibodies, the profile of IgG class antibodies and cytokin profile. In order to determine cellular immunity, lymphocyte cytotoxicity assays are used. To date, several liver fluke antigens have been used as vaccines (Wedrychowicz and Klockiewicz, 1994, Acta Parasitologica, 39:173). These include cysteine proteases. (Piacenza L. et al., 1998, Parasitol. Int., 47S:266).

Cysteine proteases are enzymes which play a significant role in host-parasite interactions. They take part in the parasite's feeding, its migration through the hosts's tissues, as well as in defence against the defensive mechanisms of the host's immune system. It is also known that during the long-term migration of the various developmental forms of the parasite in the host organism, an immune response is induced against the cysteine protease.

Kofta et al. used cysteine protease cDNA as a vaccine against liver fluke infections (Kofta et al., 2000, Vaccine, 18: 2985). Excellent results were obtained from this experiment.

Patent descriptions US6281345 (published 2001.08.28) and US6265198 (published 2001.07.24) describe pharmaceutical compositions containing nucleic acids, proteins, antibodies and inhibitors and their application in protecting animals against diseases caused by gastrointestinal parasites.

Patent description US6066503 (published 2000.05.23) describes a solution relating to nucleotide sequences coding enzymes, aminopeptidases of gastrointestinal parasites, their antigens and functional equivalents, and their application as a vaccine aginst these parasites.

In patent description US5885814 (published 1999.03.23) a vaccine is presented against parasites of the gastrointestinal tract, containing a liver fluke serine protease.

Patent descriptions US6419923 (published 2002.07.22), US6365392 (published 2002.04.02), US5795768 (published 1998.08.18), US5792624 (published 1998.08.11), US5750391 (published 1998.05.12) and US5691186 (published 1997.11.25) describe methods for the production of cysteine protease proteins, compounds which inhibit the nematode cysteine protease, and their application in pharmaceutical compositions for the protection of animals against diseases caused by gastrointestinal parasites. Isolated, recombinant cells udergo expression.

Patent description US5863775 (published 1999.01.26) describes a method for combating gastrointestinal parasites in animal hosts based on the oral administration to the animal-host of an anti-parasite protein in the form of a medication or food, such that the protein reaches the parasite. The anti-parasite protein may be an inhibitor of an enzyme of the parasite, for example an inhibitor of a digestive enzyme such as an inhibitor of cysteine protease. According to this solution, the anti-parasite protein is a protein undergoing expression in transgenic maize and rice, which are the feed for the host animals.

To date, there have been no references in literature to immunisation against *F. hepatica* invasions through the oral administration of liver fluke antigens in the form of inclusion bodies.

The Hepatitis type B virus causes severe and chronic type B hepatitis and hepatic-cellular cancer (cited in Blumberg, 1997). HBV infections in people are accomPolish Academy of Sciencesied by the intensive and maintained detectable production of antibodies stimulated by the presence of the viral nucleocapsid (HBcAg) (Milich and McLachlan 1986, Milich et al., 1997a). The viral nucleocapsid is composed of repeating subunits of its structure, dimers of the capsid protein (Zhou and Standring, 1992), which is composed of 183 or 185 amino-acids depending on the immunological subtype of the virus (Tiollais et al., 1981). Two domains are found in the capsid protein. One is responsible for the cooperative process of nucleocapsid creation, and the other, a protoamine, located on the C-end of the amino-acids (ca. 40 C-end amino-acids) responsible for nucleic acid binding.

The HBcAg protein is self-folding (Gallina et al., 1989; Birnbaum and Nassal, 1990). *In vivo* it agglomerates into capsids containing pre-genomic viral RNA and a DNA polymerase encoded by HBV (Bartenschlager and Schaller, 1992). The protein possesses nuclear translocation signals in its C-end domain (Yeh et al., 1990; Eckhardt et al., 1991).

To create capsid molecules, the first 140 N-terminal amino-acids suffice, wherein proline 138 is essential (Metzger and Bringas, 1998). In several laboratories a capsid structure has been determined, which in solution are composed of shortened sections of the core protein. (Crowther et al., 1994; Bottcher et al., 1997; Conway et al., 1997). It turns out that the capsid is formed from dimers of the core protein (see also Zhou and Standring, 1992). It is thought that the dominant form is amolecule composed of 120-dimers (Bottcher et al., 1997; Conway et al., 1997), which expose amino-acids 78-83 to the outside(Conway et al., 1998b). From the models presented by (Bottcher et al., 1997; Conway et al., 1997; Conway et al., 1998a; Zlotnick et al., 1997), however, it is shown that the C- and N-ends of the core proteins, forming the capsid from shortened core proteins, are also accessible from the outside. Capsid structure is dependent to a degree on the number of amino-acids of the core protein, which were removed from the C-end (Zlotnick et al., 1996). The mentioned characteristics are significant in the construction of hybrid capsids bearing additional antigens.

It has been shown that HBcAg possesses a unique capability of inducing immune response, despite being an internal component of the HBV virion. During many chronic infections, HBcAg is the only antigen capable of inducing an immune response. It is known that it is enough to administer 25 ng of this antigen in an injection without any adjuvant to stimulate the production of antibodies (Milich et al., 1997b). The work of Milich and his colleagues, performed on mice have shown that the large immunogenic activity of HBcAg stems from the ability of this antigen to activate Bcells to produce antibodies directed against HBcAg, both in the presence of specific T cells, and without them (Milich and McLachlan, 1986). HBcAg-specific B cells from non-immunised mice perform the function of primary antigen-presenting cells, since they process and present the antigen to naive T cells *in vivo,* more actively than macrophages and dendritic cells (Milich et al., 1997a). HBcAg also effectively immunises T cells, particularly Th1 (Milich et al., 1987a).

Furthermore, it was determined that helper cells specific for HBcAg can interact with type B cells, specific for the surface antigen (HBsAg), stimulating them to produce appropriate antibodies (Milich et al., 1997b).

These unique properties are possessed by nucleocapsid molecules, and not secreted forms of the antigen (HBeAg).

Fusion to the immunodominant internal domain of HBcAg causes a loss of the immunogenic and antigenic activity of HBcAg, while significantly stimulating that of the introduced foreign epitope (Schodel et al., 1990a; Clarke et al., 1991).

## THE GOAL OF THE INVENTION

Despite the above described preliminary studies on producing a vaccine against the liver fluke, a real need still exists to produce effective tools for the easy production of effective vaccines immunising against this parasite.

In the light of the above described state of technology, the goal of the present invention is to deliver means which are effective and easily obtainable enough, such that they may be used in oral vaccination against the liver fluke, which would facilitate the acquisition of systemic immunity as well as mucous membrane immunity. The means according to the present invention should be easy to apply and lacking the other known disadvantages connected with the preparation and application of vaccines, such as the relatively high cost of preparation of the medication, and the possible danger of blood contamination during immunisation. Oral vaccines according to the present invention should ensure effective immunisation through the gastrointestinal tract.

The next goal of the invention is the delivery of means which could be used in the production of an effective, easy to apply and easily available vaccine against the liver fluke. A particular goal of the invention is the production of chimaeric proteins, which would contain antigens of the cysteine protease of *Fasciola hepatica* and could be used to vaccinate against the liver fluke, and also tools facilitating their expression in *E. coli.* A particular goal of the invention is the production of means for the production of recombined proteins, which could be applied in the production of edible vaccines against the liver fluke.

Unexpectedly, the embodiment of such a described goal and a solution of the problems described in the state of technology, connected with proteins forming on the basis of exogenous DNA have been met in the present invention.

## SUMMARY OF THE INVENTION

The first aspect of the invention are inclusion bodies containing a polypeptide produced in a genetically modified microorganism, containing the amino-acid sequence of the liver fluke cysteine protease sequence, possibly fused to the amino-acid sequence of the HBV core protein, for use as an oral vaccine against the liver fluke. Preferably, they are produced in *E.coli.* Also preferably, the polypeptide contains one of the following sequences:

- the sequence of the catalytic domain of the liver fluke cysteine protease fused to the sequence of the HBV core protein,
- the sequence of the leading domain of the liver fluke cysteine protease fused to the sequence of the HBV core protein,

- the sequence of the catalytic domain of the liver fluke cysteine protease fused to the sequence of the HBV core protein and glycine residues, forming a glycine bridge structure,
- the sequence of the catalytic domain of the liver fluke cysteine protease,
- the sequence of the precursor protein of the liver fluke cysteine protease encompassing the catalytic and leading domains.

Particularly preferably, the polypeptide contains one of the sequences encoded by the sequences presented in Figures 7 to 15.

The next aspect of the invention is use of a polypeptide in the form of inclusion bodies containing the amino-acid sequence of the sequence of the liver fluke cysteine protease, possibly fused to the amino-acid sequence of the HBV core protein, in the preparation of a vaccine against the liver fluke. Preferably, the polypeptide is produced in a genetically modified microorganism. Also preferably, the polypeptide contains one of the following sequences:

- the sequence of the catalytic domain of the liver fluke cysteine protease fused to the sequence of the HBV core protein,
- the sequence of the leading domain of the liver fluke cysteine protease fused to the sequence of the HBV core protein,
- the sequence of the catalytic domain of the liver fluke cysteine protease fused to the sequence of the HBV core protein and glycine residues, forming a glycine bridge structure,
- the sequence of the catalytic domain of the liver fluke cysteine protease,
- the sequence of the precursor protein of the liver fluke cysteine protease encompassing the catalytic and leading domains. Particularly preferably, the polypeptide contains one of the sequences encoded by the sequences presented in Figures 7 to 15.

  The next aspect of the invention is also an oral vaccine against the liver fluke containing an antigen and possibly a pharmaceutically acceptable carrier and/or adjuvant, characterised in that as the antigen it contains a polypeptide in the form of inclusion bodies as defined above.

  In the present invention, HbcAg was used as a carrier and adjuvant for the antigen of the catalytic domain of the liver fluke cysteine protease.

  Two construction variants of constructs introduced into the expression vectors were used:

    1. Fusion of the 5’ end of the sequence coding the HBV core protein with the 3’ end of the sequence coding an appropriate fragment of the liver fluke cysteine protease,
    2. Fusion of the fragment of the sequence coding the catalytic domain of the liver fluke cysteine protease with the sequence coding the HBV core protein through a "glycine bridge". This type of bond, according to the Kratz article P.5, causes the protein introduced into the HBV core protein to be exposed on its surface.

Vaccines are appropriate proteins, whose expression has been achieved in *E. coli* bacterial cells of the strain BL21 (DE3) transformed with the pIGCmT7 expression plasmid containing a given insert. These proteins were isolated from the E. *coli* bacteria in the form of inclusion bodies.

## THE VIRTUES OF THE INVENTION

The present invention possesses many advantages setting it apart from solutions known from the state of the technology. An extremely effective vaccine against the liver fluke, has been obtained, which facilitates systemic immunity, and also mucous membrane immunity.

Due to the fact that the natural invasion pathway of the liver fluke is the gastrointestinal tract, a vaccine has been produced for oral administration, which induces an effective immune response at the natural flatworm penetration site. The vaccine produced is easy to apply and lacks the other known disadvantages connected with the preparation and application of classical vaccines, such as the high cost of preparation of the medication, and the danger of accidental blood contamination during the vaccination.

The antigen expression occurs in bacterial cells. The simple design of the host and the ability to control the process of the expression of the vaccine antigen isolated in the form of inclusion bodies guarantees the homogeneity of the prepared medicaments, and thus precise dosage control.

## BRIEF DESCRIPTION OF THE DRAWINGS

The attached figures facilitate a better understanding of the nature of the present invention.

Figure 1 represents a map of the pIGCmT7 plasmid, where ARG t-RNA is the gene for arginine tRNA for the AGA and AGG codons, ORI is origin of replication of the pPIGDM1 plasmid, Cm-R is the chloramphenicol resistance gene, T7 is a promoter, stop is a transcription termination signal;

Figure 2 represents the nucleotide sequence of the whole liver fluke cysteine protease *(Fasciola hepatica);*

Figure 3 represents the isolated protein of the catalytic domain of the liver fluke cysteine protease; Lanes: 1) Protein mass standard LMW 14,4 kDa, 20,1 kDa, 30,0 kDa, 43,0 kDa, 67,0 kDa, 94,0 kDa, 2) Isolated protein of the catalytic domain of the liver fluke cysteine protease.

Figure 4 represents the isolated precursor protein of the liver fluke cysteine protease (both the catalytic and leading domains); Lanes: 1) Protein mass standard LMW 14,4 kDa, 20,1 kDa, 30,0 kDa, 43,0 kDa, 67,0 kDa, 94,0 kDa, 2) Isolated precursor protein of the liver fluke cysteine protease (both the catalytic and leading domains).

Figure 5 represents the isolated protein of the catalytic domain of the liver fluke cysteine protease::fragment of the HBV core domain; Lanes: 1) Protein mass standard LMW 14,4 kDa, 20,1 kDa, 30,0 kDa, 43,0 kDa, 67,0 kDa, 94,0 kDa, 2) Isolated protein of the catalytic domain of the liver fluke cysteine protease::fragment of the HBV core domain.

Figure 6 represents isolated protein of the catalytic domain of the liver fluke cysteine protease::glycine bridge:: fragment of the HBV core domain. Lanes: 1) Protein mass standard LMW 14,4 kDa, 20,1 kDa, 30,0 kDa, 43,0 kDa, 67,0 kDa, 94,0 kDa; 2) Isolated protein of the catalytic domain of the liver fluke cysteine protease::glycine bridge:: fragment of the HBV core domain.

Figure 7 represents the CWekGlyKatwBluescr sequence coding the hybrid protein HBV core domain:: catalytic domain of the liver fluke cysteine protease, connected by a glycine bridge. The fragment was inserted into a Bluescript SK (-) vector into sites created by digestion with the *Nde*I and *Hind*III restriction nucleases.

Figure 8 represents the CWekGlyKatwPIG sequence coding the hybrid protein HBV core domain::catalytic domain of the liver fluke cysteine protease, connected by a glycine bridge. The fragment was inserted into a pIGCmT7 vector into sites created by digestion with the *Nde*I and *Hind*III restriction nucleases.

Figure 9 represents the Moty_cat_Ckrd sequence coding the hybrid protein HBV core domain::catalytic domain of the liver fluke cysteine protease. The fragment was inserted into a Bluescript SK (-) vector into sites created by digestion with the *Bam*HI and *Sac*I restriction nucleases.

Figure 10 represents the KAT_Nasza_MOT1_MOT2 sequence coding the catalytic domain of the liver fluke cysteine protease. The fragment was inserted into a Bluescript SK (-) vector into sites created by digestion with the *Bam*HI and *Hind*III restriction nucleases.

Figure 11 represents the sequence coding the catalytic domain of the liver fluke cysteine protease. The fragment was inserted into a pIGCmT7 vector into sites created by digestion with the *Nde*I and *Hind*III restriction nucleases.

Figure 12 represents the MOT_DO_C_POP_MOT1_MOT3_W_BLUESCRIPCIE sequence coding the catalytic domain of the liver fluke cysteine protease. The fragment was inserted into a Bluescript SK (-) vector into sites created by digestion with the *Bam*HI and *Hind*III restriction nucleases.

Figure 13 represents the MOT_DO_C_POP_MOT1_MOT3_W_PIG sequence coding the catalytic domain of the liver fluke cysteine protease. The fragment was inserted into a pIGCmT7 vector into sites created by digestion with the *Nde*I and *Hind*III restriction nucleases.

Figure 14 represents the Moty_cat_Ckrd_w_PIG sequence coding the hybrid protein HBV core domain::catalytic domain of the liver fluke cysteine protease. The fragment was inserted into a pIGCmT7 vector into sites created by digestion with the *Nde*I and *Hind*III restriction nucleases.

Figure 15 represents the Cala_Mot_N sequence coding the precursor protein of the liver fluke cysteine protease (both the catalytic and leading domain). The fragment was inserted into a pIGCmT7 vector into sites created by digestion with the *Nde*I and *Hind*III restriction nucleases.

The following figures represent the results of serological analyses in animals orally inoculated with medications according to the present invention. Figures 16-20 represent the levels of antibodies of the following classes: IgG1, IgG2a, IgG2b, IgE and IgA reacting with PC or ES of the liver fluke, where Figure 16 represents the level of IgG1 antibodies specific for PC; Figure 17 represents the level of IgG2a antibodies specific for PC, Figure 18 represents the level of IgG2b antibodies specific for PC, Figure 19 represents the level of IgE antibodies specific for PC, a Figure 20 represents the level of IgA antibodies specific for PC.

Below are example embodiments, as defined above in the present invention.

**Example 1**. Production of a strain of *E. coli* expressing the vaccinating antigens.

Plasmids

The bacterial expression plasmid, pIGCmT7: Plasmid pIGCmT7 (4056 bp.) (Fig.1) was created in the laboratory of Prof. Andrzej Plucienniczak at the Institute of Biotechnology and Antibiotics in Warsaw based on plasmid pIGDM1 (Mikiewicz D. et al., Plasmid 1997), which is a plasmid of the ColE1 group of *Enterobacter agglomerans.* Plasmid pIGCmT7 was created by the addition of a chloramphenicol resistance gene, a polylinker along with the T7 phage RNA polymerase promoter, and a sequence coding the transcription stop codon from the T7 phage.

Non-expression bacterial plasmids: To clone fragments following PCR amplification the pBluescript SK(-) plasmid from Stratagene was used (the sequence is available from Gene Bank, sccession No. X52324 S52394). This plasmid possesses the following: a Col E1 origin of replication, T3 and T7 phage RNA polymerase promoters flanking a polylinker and an ampicylin resistance gene.

Initial insert sequences

The sequence of the liver fluke cysteine protease (*Fasciola hepatica):* the cDNA of the whole liver fluke cysteine protease *(Fasciola hepatica)* was isolated at the Institute of Biotechnology and Antibiotics, in cooperation with the Chair of Parasitology of the Agricultural University. The work was performed on a strain of *Escherichia coli* based on a plasmid bearing the gene coding the cysteine protease protein of *Fasciola hepatica* (Kofta W, Mieszczanek J, Plucienniczak G, Wedrychowicz H., Successful DNA immunisation of rats against fasciolosis, Vaccine 2000; 18:2985-2990).

A comparison to other protease sequences gathered at the Gene Bank became the basis for the differentiation of three coding regions in the nucleotide sequence of the protease: the signal, leading and catalytic fragment.

The nucleotide sequence of the entire liver fluke cysteine protease (*Fasciola hepatica*) is attached Fig.2.

The sequence coding the hepatitis type B virus core protein (HBV) of the strain *ayw*4*:* The nucleotide sequence coding the HBV core protein (*ayw*4) was isolated by Prof. Plucienniczak. It is available from the Gene Bank, accession No. Z35716. A fragment of DNA coding the HBV core protein (*ayw*4) cloned into the HBV 321 plasmid. This is a PUC19 plasmid, containing the whole genome of HBV *ayw*4 inserted into the sites created by digestion with the *Bam*HI restriction nuclease.

Schematics of the production of vectors with inserts.

The coordinates given in the descriptions of the production of vectors with inserted fragments refer to respectively to the coordinates in the attached sequence in the case of the liver fluke cysteine protease and of the sequence deposited in the American Gene Bank in the case of HBV *ayw*4.

Schematics of the production of the KAT_Nasza_MOT1_MOT2 fragment inserted into the pIGCmT7 vector.

From the plasmid containing the full nucleotide sequence of the liver fluke cysteine protease, a PCR reaction was used to amplify a fragment of DNA coding the C-terminal (catalytic) domain of this protease, from position 325 to 982. In the PCR, the MOT1 forward primer and MOT2 reverse primer were used. The primers used in the PCR were designed based on the sequence of the DNA coding the protease. The primers introduced restriction nuclease recognition sites, *Bam*HI and *Nde*I for the forward primer, and *Hind*III for the reverse. The amplified fragment was separated electrophoretically and isolated from the polyacrylamide gel. (Dybczyński I, Phucienniczak A, A protocol for DNA fragment extraction from polyacryloamide gels, Biotechniques 1988; 6:924-926), and then digested with the *Bam*HI and *Hind*III restriction nucleases and deproteinated. The DNA fragments produced were ligated with a pBluescriptSK(-) vector digested with the same restriction nucleases. The ligation product was transformed into *E. coli* cells of the strain NM522. The transformation method used is described by J. Sambrook et al. Chung C. T., Miller R. H., A rapid and convenient method for the preparation and storage of competent bacterial cells, Nucleic Acid. Res. 1988;16(8). Following the isolation of plasmid DNA from the transformed *E. coli* cells, the presence of the cloned KAT_Nasza_MOT1_MOT2 insert was confirmed through restriction analysis, and the accuracy of the sequence was verified by sequencing. The fragment was digested out of the pBluescriptSK(-) vector using the *Nde*I and *Hind*III restriction enzymes, and deproteinated. The DNA fragment produced was ligated with the pIGCmT7 vector, digested with the same restriction enzymes, and deproteinated following digestion. The ligation product was used to transform *E. coli* cells of the strain NM522. The presence of the cloned KAT_Nasza_MOT1_MOT2_w_PIG insert in the pIGCm T7 vector was confirmed by restriction analysis.

A plasmid containing the confirmed insert was transformed into *E. coli* cells of the strain BL21(DE3).

Schematics of the production of the CWekGlyKatwPIG fragment inserted into the pIGCmT7 vector

Stage I: PCR with the primers COREWP and COREWK was used to amplify a fragment of the gene coding the HBV *ayw*4 core domain (from position 1903 to 2349) from the HBV321 plasmid containing the entire genome of HBV *ayw*4.. The primers used in the PCR were designed based on the sequence of the DNA coding the HBV *ayw*4 core domain. The leading primer, COREWP, elongates the DNA molecule at the 5', introducing recognition sites for the restriction nucleases *Eco*RI and *Nde*I*.* It also introduces changes in the nucleotide sequence, exchanging cytosines 1908 and 1914 for thymines, introducing a recognition site for the *Cla*I restriction nuclease, while maintaining the aminoacid sequence. The reverse primer, COREWK, elongates the DNA molecule at the 3' end, and introduces a recognition site for the *Sac*I and *Hind*III restriction nucleases, as well as a TAA termination codon. Following PCR, the mixture was separated electrophoretically on a polyacrylamide gel. The amplified DNA fragment was eluted from the polyacrylamide gel and then digested with the *Eco*RI and *Hind*III restriction enzymes, and deproteinated. The DNA fragment produced was ligated to the pBluescript SK(-) vector digested with the same restriction enzymes and deproteinated following digestion. The ligation product was used to transform E. *coli* cells of the strain NM522. Following the isolation of plasmid DNA, the presence of the insert was confirmed by restriction analysis. From the pBluescript SK(-) vector containing the cloned insert, a fragment was digested out using the *Hind*III and *Bgl*II enzymes. The post-digestion mixture was separated electrophoretically on a agarose gel, from which the fragment corresponding to the pBluescript SK(-) vector containing the 97 bp HBV *ayw*4 fragment was excised and eluted.

Stage II: The HBV *ayw*4 core domain fragment was amplified from the HBV 321 plasmid containing entire genome of HBV *ayw*4 using PCR, with the primers CXBAD and COREWK. Both primers were designed based on the sequence of the DNA coding the HBV *ayw*4 core domain. The reverse primer, COREWK introdueces restriction sites as described above into the sequence of the HBV *ayw*4 core domain fragment. Primer CXBAD was designed based on the sequence coding the HBV ayw4 core domain fragment. This primer ten exchanges adenine 1995 for guanine, eliminating a recognition site for the *Xba*I restriction nuclease, while maintaining the amino-acid sequence. Following the PCR the mixture was separated electrophoretically on a polyacrylamide gel. The amplified DNA fragment was eluted out of the gel and then digested with the *Hind*III and *Bgl*II restriction nucleases and then deproteinated.

Stage III: DNA fragments were ligated, which were the end products of procedures described in Stage I and II. The ligation product was used to transform *E. coli* cells of the strain NM522. Following the isolation of plasmid DNA from the transformed colonies of *E. coli,* the presence of the insert was confirmed using restriction analysis. The isolated plasmid was used as a template in two PCR reactions:

Using the primers COREWP (forward) and CXHOBAM (reverse). The reverse primer was designed based on the sequence of HBV *ayw*4 from position 2116 to 2142. This primer introduces the following changes into the nucleotide sequence: thumine 2128 is exchanged for cytosine, guanine 2130 is exchanged for cytosine, and adenine 2133 is exchanged for guanine.

The changes introduced maintain the amino-acid sequence and introduce recognition sites for the restriction nucleases *Xho*I and *Bam*HI.

Using the primers CBAMAVR (forward) and COREWK (reverse). The leading primer was designed based on the sequence of HBV *ayw*4 from position 2120 to 2151. This primer introduces the same changes as primer CXHOBAM and additionally exchanges thymine 2143 for cytosine. The changes introduced using primer CXHOBAM facilitate the digestion of the DNA fragment, on the stretch on which it is complementary, with the restriction nucleases *Xho*I*, Bam*HI and *Avr*II.

The products of the above PCR reactions were separated electrophoretically on a polyacrylamide gel.

In the first PCR, a DNA fragment of 251 bp. was amplified, and in the second PCR, a DNA fragment of 249 bp. was amplified. Both fragments were eluted from the polyacrylamide gels. The mixtures of both fragments were used as templates for PCR using the primers COREWP and COREWK. The mixtures from both PCRs were separated on polyacrylamide gels. The amplified DNA fragment of 477 bp. in length was isolated from the polyacrylamide gel through elution and then digested with the restriction enzymes *Nde*I and *Hind*III and deproteinated. The DNA fragment produced was ligated with the pIGCmT7 vector digested with the same restriction nucleases. The ligation product was used to transform *E. coli* cells of the strain NM522.

Stage IV: Preparation of the "glycine bridge", a DNA fragment cloned into sites arising from digestion with the restriction enzymes *Bam*HI and *Avr*II in the construct obtained in Stage III.

Phosphorylation of the oligonucleotide SCORE2 using bacteriophage T4 polynucleotide kinase.

Phosphorylation of the oligonucleotide SCORE3 SCORE2 using bacteriophage T4 polynucleotide kinase.

Ligation of the phosphorylated oligonucleotide SCORE2 with oligonucleotide SCORE4 using the complementary portions of the sequence.

Ligation of the phosphorylated oligonucleotide SCORE3 with oligonucleotide SCORE1 using the complementary portions of the sequence.

Ligation of products obtained in points 3. and 4.

The ligation mixture was separated electrophoretically in a polyacrylamide gel. The DNA froagment of 73 bp. was isolated from the polyacrylamide gel through elution. The eluted DNA fragment was phosphorylated and then ligated with the vector obtained in Stage III. The vector was digested with restriction nucleases *Avr*II and *Bam*HI*,* and deproteinated prior to the ligation. The ligation product was used to transform *E. coli* cells of the strain NM522. following the isolation of plasmid DNA from the *E. coli* culture transformed with the vector, the presence of the cloned insert was confirmed using restriction analysis.

Stage V: Using PCR, the fragment coding the catalytic domain sequence of the liver fluke cysteine protease from position 325 to 982 was amplified from the plasmid containing the full sequence of this enzyme. In this PCR the primers MOTEPW (forward) and MOTEKWIN (reverse) were used. The primers were designed based on the DNA sequence coding catalytic domain of the liver fluke cysteine protease. Primer MOTEPW elongates the 5' end of the DNA molecule and introduces a recognition site for the restriction nuclease *Bgl*I and a change in the nucleotide sequence. Cytosine 327 is exchanged for thymine. The change introduced maintains the amino-acid sequence. Primer MOTEKWIN elongates the 3' end of the DNA molecule, introducing recognition sites for the restriction nucleases *Cla*I and *Bgl*I. Following the PCR the mixture was separated electrophoretically on a polyacrylamide gel. The amplified DNA fragment of 700 bp was eluted from the polyacrylamide gel, and then digested with the restriction nuclease *Bgl*I and deproteinated. The DNA fragment prepared was ligated with the vector obtained in Stage IV, digested with restriction nuclease *Sfi*I and deproteinated after digestion. The ligation product was used to transform *E. coli* cells of the strain NM522. Following the isolation of plasmid DNA from cultures transformed *E. coli,* restriction analysis was used to confirm the presence of the insert CWekGlyKatwPIG. The accuracy of the CWekGlyKatwPIG fragment cloned on the pIGCmT7 plasmid was confirmed using the primers PIGMSEK and T7SEK complementary to the polylinker sequence of plasmid pIGCmT7. In the sequencing of this construct, the primers MOTSEK1, MOTSEK4, and MOTSEK3 were used, which are complementary to the sequence of the catalytic domain of the liver fluke cysteine protease and oligonucleotide SCORE4.

A plasmid containing the confirmed insert was transformed into *E. coli* cells of the strain BL21 (DE3).

Schematic of the production of Moty_cat_Ckrd_w_PIG inserted into the pIGCmT7 vector

Stage I: Using PCR, a fragment of the gene coding the HBV *ayw*4 core domain (from position 1903 to 2349) was amplified from plasmid HBV321 containing the entire genome HBV *ayw*4*,* using the primers COREWP and COREWK. The primers used were designed based on the DNA sequence coding the HBV ayw4 core domain fragment. The forward primer COREWP elongates the DNA molecule at the 5' end, introducing rescognition sites for the restriction nucleases *Eco*RI and *Nde*I and introduces changes in the nucleotide sequence, exchanging cytosines 1908 and 1914 for thymine, and introducing a recognition site for the restriction nuclease *Cla*I, while maintaining the amino-acid sequence. The reverse primer COREWK introduces recognition sites for the restriction nucleases *Sac*I and *Hind*III and a transcription stop sequence. The PCR reaction mix was separated electrophoretically on a polyacrylamide gel. The amplified DNA fragment was eluted from the polyacrylamide gel, and then digested with the restriction ezymes *Cla*I and *Hind*III*,* and deproteinated. The DNA fragment produced was ligated to a pBluescript SK(- ) vector digested with the same restriction nucleases and deproteinated following digestion. The ligation product was used to transform *E. coli* cells of the strain NM522. Following the isolation of plasmid DNA the presence of the insert was confirmed with restriction analysis, and the accuracy of the insert was confirmed by sequencing. The produced plasmid pBluescript SK(-) containing the cloned fragment of the gene coding HBV *ayw*4 core domain was transformed into E. *coli* cells of the strain BZ37. Following the isolation of the plasmids from the cultures of transformed *E. coli* of the strain BZ37, they were digested with the restriction nucleases *Cla*I and *Hind*III. The mixture was separated electrophoretically on a polyacrylamide gel, from which a fragment of 450 bp in length was first excised and then eluted.

Stage II: Using PCR, the gene coding catalytic domain of the liver fluke cysteine protease from position 324 to 982 was amplified from the plasmid containing the complete nucleotide sequence of this protease. In the PCR, the following primers were used: MOT1 (forward) and MOT3 (reverse). The rpimers were desgined based on the DNA sequence coding the catalytic domain of the liver fluke cysteine protease. Primer MOT1 elongates the 5' end of the DNA molecule, introducing recognition sites for the restriction nucleases *Bam*HI and *Nde*I*,* and a change in the nucleotide sequence. Cytosine 327 is exchanged for thymine. The change introduced maintains the amino-acid sequence. Primer MOT3 elongates the 3' end of the DNA molecule, introducing recognition sites for restriction nucleases *Hind*III and *Cla*I, and the termination codon TAA. Following the PCR, the mixture was separated elecrophoretically on a polyacrylamide gel. The 688 bp long fragment was eluted from the polyacrylamide gel, and the digested with the restriction nucleases *Bam*HI and *Hind*III and deproteinated. The DNA fragment produced was ligated with a pBluescript SK(-) vector digested with the same restriction nucleases and deproteinated following the digestion. Following the isolation of plasmid DNA from the transformed *E. coli,* the presence of the insert MOT_DO_C_POP_MOT1_MOT3_W_BLUESCRIPCIE was confirmed using restriction analysis. From the pBluescript SK(-) vector containing the cloned insert, a DNA fragment was digested out using the restriction enzymes *Nde*I and *Hind*III*.* The digestion reaction mixture was separated electrophoretically on a polyacrylamide gel, from which a 679 bp fragment was first excised and then eluted. The DNA fragment obtained was ligated to the pIGCmT7 vector digested with the same restriction nucleases and deproteinated following the digestion. The ligation product was used to transform *E. coli* cells of the strain NM522. Following the isolation of plasmid DNA from ccolonies of transformed *E. coli,* restriction analysis was used to confirm the presence of the insert MOT_DO_C_POP_MOT1_MOT3_W_BLUESCRIPCIE cloned into the pIGCmT7 vector. Next, the produced vector was digested with the restriction nucleases *Cla*I and *Hind*III. The digestion reaction mixture was separated electrophoretically in an agarose gel, from which a fragment corresponding to the pIGCmT7 vector with a 678 bp fragment was first excised, and then eluted.

Stage III: DNA fragments were ligated which were the end products of procedures described in Stages I and II. The ligation product was used to transform *E. coli* cells of the strain NM522. Following the isolation of plasmids from the cultures of transformed *E. coli* cells, the presence of the cloned MOTY_cat_Ckrd_w_PIG insert in the pIGCmT7 vector was confirmed using restriction analysis.

A plasmid containing the confirmed insert was used to transform *E. coli* cells of the strain strain BL21(DE3).

Schematic of the production of the fragment Cala_Mot_N inserted into the expression vector pIGCmT7.
Using PCR a fragment coding the precursor of the liver fluke cysteine protease (both the catalytic and leading domains) was amplified from position 52 to 982, from the plasmid containing the full nucleotide sequence of this protease. In the PCR the primers MOTPRO1 (forward) and MOT3 (reverse) were used. The primers were designed based on the DNA sequence coding the precursor of the liver fluke cysteine protease. Primer MOTPRO1 elongates the 5' end of the DNA molecule, introducing recognition sites for the restriction nucleases *Bam*HI and *Nde*I. Primer MOT3 elongates the 3' end of the DNA molecule, introducing recognition sites for the restriction nucleases *Hind*III and *Cla*I, and the termination codon TAA. Following the PCR, the mixture was separated electrophoretically on a polyacrylamide gel. The amplified fragment, 960 bp long, was eluted from the polyacrylamide gel, and then digested with the restriction nucleases *Bam*HI and *Hind*III and deproteinated. The produced DNA fragment was ligated to the pBluescript SK(-) vector digested with the same restriction nucleases and deproteinated following the digestion. The ligation product was used to transform *E. coli* cells of the strain DH5α. Following the isolation of plasmid DNA from transformed bacteria, restriction analysis was used to transform the presence of the Cala_Mot_N insert in the vector pBluescript SK(-).
From the pBluescript SK(-) vector containing the insert, a fragment was digested using the restriction enzymes *Nde*I and *Hind*III DNA. The digestion mixture was separated electrophoretically on a polyacrylamide gel, from which a fragment of a length corresponding to the Cala_Mot_N insert was first excised, and then eluted. The produced DNA fragment was ligated to the pIGCmT7 vector digested with the same restriction nucleases and deproteinated following the digestion. The ligation product was used to transform cells of *E. coli* of the strain DH5α. Following the isolation of plasmid DNA from cultures of transformed *E. coli,* restriction analysis was used tconfirm the presence of the Cala_Mot_N insert in the pIGCmT7 vector. The accuracy of the sequence of the Cala_Mot_N fragment inserted into the pIGCmT7 vector was verified by sequencing.

Transformation of *E. coli* cells

The transformation of competent *E. coli* bacterial cells method was used, as described przez J. Sambrook et al. (*supra*).

*Preparation of inclusion bodies and vaccine antigens.*

Expression and isolation of inclusion bodies

*E. coli* strain BL21(DE3) bacteria carrying an appropriate recombined plasmid were cultured on an LB medium containing chloramphenicol (20μg/ml) at 37°C for 3 hours, after which it was diluted 1:100 with fresh LB medium, and following another three hours of shaking at 37°C expression was induced by the addition of isopropyl-thio-β-D-galactoside (IPTG) to a final concentration of 0,1 μg/ml. After 3 hours the bacteria were centrifuged down. The cell suspension was suspended in lysis buffer (0.5 M NaCl, 0.05 M Tris HCl, 0.01 M EDTA, 0.005 M 2-Mercaptoethanol, 0.35 mg/ml) and incubated for 30 at 20°C. Triton X-100 was added to a final concentration of 1%. The suspension was sonificated and centrifuged. The inclusion body sediment was suspended twice in PBS buffer containing 1% Triton X-100, sonificated and centrifuged. The isolated inclusion bodies were washed twice with PBS. Finally the inclusion bodies were suspended in PBS buffer.

Analysis of the purified inclusion bodies

The presence of the inclusion body fraction was confirmed by analyzing the samples electrophoretically in a 15% polyacrylamide gel (SDS-PAGE). Proteins were visualised with the Coomassie blue stain.
Images of the electrophoretic separations of samples of isolated inclusion bodies are presented in Figures 3, 4, 5 and 6. Spectrophotometric determination of protein concentration
The concentration of proteins was determined by measuring absorbance at λ=595 nm using Bradford reagent.

Primers and PCR oligonucleotides.

Primers used to amplify DNA fragments were ordered from and made by the Perkin Elmer comPolish Academy of Sciencesy.

Name: COREWP
5'-GGG GAA TTC ATA TGG ATA TCG ATC CTT ATA AAG AAT TTG GA-
Name: COREWK
5' -TCT CGA GCT CAA GCT TTT AAA CAA CAG TAG TCT CCG G-

Name: MOT1
5' -GGG GAT CCA TAT GGC TGT ACC CGA CAA AAT TGA C-
Name: MOT2
5' -AAA AGC TTA ATC GAT ATC ACT ACC GCT ACC CGG AAA TCG TGC CAC CAT C-
Name: MOT3
5' -AAA AGC TTA ATC GAT GCC GGA AAT CGT GCC-
NAME: SEQBLT3
5' -AAA TTA ACC CTC ACT AAA GG-
Name: MOTPRO1
5' -GGG GAT CCA TAT GTC GAA TGA TGA TTT GTG G-
Name: CXBAD
5' -GTA CGA GAT CTT CTG GAT AC-
Name: CXHOBAM
5' -TGC TGG ATC CTC GAG ATT AAC ACC CAC-
Name: CBAMAVR
5' -GTG TTA ATC TCG AGG ATC CAG CAC CTA GGG AC-
Name: SCORE1
5' -GAT CAA GGT GGT GGA GGT TCT GGT GGA GGA GGC CAA GG-
Name: SCORE2
5' -AGG CCA AGG AGG AGG TGG TTC AGG CGG TGG TGG AT-
Name: SCORE4
5' -CTA GAT CCA CCA CCG CCT GAA CCA CCT CCT CCT TGG CCT CCT-
Name: MOTEPW
5' -AAA GGC CAA GGA GGC GCT GTA CCC GAC AAA ATT GAC-
Name: MOTEKWIN
5' -AAA GGC CTC CTT GGC CAT CGA TGC CGG AAA TCG TGC C-
Name: SCORE3T
5' -TGG CCT CCT CCA CCA GAA CCT CCA CCA CCT T-
Name: MOTSEK1
5' -CCC GAC AAA ATT GAC TGG CG-
Name: MOTSEK2
5' -GGC AAC TCC TAA CTG CTT ATT G-
NAME: MOTSEK3
5' -GGA TTG TGA AAA ATA GTT GGG G-
Name: MOTSEK4
5' -CCC CAA CTA TTT TTC ACA ATC C-
Name: T7SEK
5' -CCA CAA CGG TTT CCC TCT AGA AA-
Name: PIGMSEK
5' -CGG TGG CAG CAG CCA ACT CA-

Apparatus and other materials:

Enzymes used in the cloning: Taq DNA polymerase and buffer (Roche, ExPolish Academy of Sciencesd Long Template PCR System kit, Cat. No. 1 681 834); T4 phage DNA ligase and buffer (USB); T4 polynucleotide kinase and buffer (USB)

Commercially available restriction nucleases were used.

PCR: the PCR thermocycler was a PTC-100™ Programmable Thermal Controller MJ Research, Inc.

Sequencing: the sequencer used was supplied by Visible Genetics, Inc.; a kit from Amersham Cat. No. US79840 (according to the manufacturer's recommendations); Plasmid DNA purification kit by Kucharczyk Techniki Elektroforetyczne.

Protein concentration determination: Philips PU 8730 spectrophotometer.

Documentation: Sony Digital Graphics Printer UP 0890 gel documentation kit and *Grab it* software.

Bacterial strains: strains of E. *coli* used in the work:

NM522 *supE thi* Δ *(lac-proAB) hsd5 F'[proAB*$^+$ *lacI*$^q$ *lacZ*Δ *M15]*
DH5α *supE44* Δ*lacU169* (φ80 *lacZ*Δ*M15) hsdR17 recA1 endA1 gyrA96 thi-1 relA1*
BL21(DE3) *hsdS gal* (λcIts857 *ind*1 Sam7 *nin5 lacUV*5-T7 gene 1)

BZ37 *dam⁻ dcm⁻*

The sequences of the inserts introduced into the vectors are presented in Figures 7-15.

In the sequences, the recognition sites for restriction enzymes are underlined. Following the digestion with these enzymes, the given insert was introduced into a vector. The start and termination codons are marked in boldface.

**Example 2**. Analysis of the efficacy of *F. hepatica* cysteine protease expressed in the form of inclusion bodies in the immunisation of a host against invasions of this parasite.

The experiment was performed on rats, since the immune and defensive response of these animals is similar to the bovine, the most common host for *F. hepatica.*

Rats of the Sprague Dawley strain (an inbred strain) were purchased from the Department of Laboratory Animals of the Institute Centre for Polish Mothers' Health, ul. Rzgowska 281/289, Lódż.

The experiments were performed with the permission of the III Local Ethics Commission in Warsaw. The rats were put into groups of 4 individuals of the same sex, in standard plastic cages placed in a climate-controlled cabinet with HEPA filtered air, in natural photoperiod conditions. The rats were given water from 750 ml bottles (Tecniplast) and fed *ad libitum* with LSM diet.

Prior to the experiment the rats were acclimatised for 1 to 2 weeks. A parasitological analysis of the faeces was performed at this time, to determine any other invasions which might influence the course of the experiment.

In the faeces studied, no developmental forms of parasites were noted.

None of the rats died during the experiment, and the rats in the physiological control group demonstrated stability of haematological indicators, which would indicate that they did not undergo any serious infections.

The *in vivo* collection of small volumes of blood (150 μl each time) during the course of the experiment was performed by incising the tip of the tail

During the day of the sections, 5ml of blood was removed from the hearts of the rats under deep narcosis. Following the collection, the anaesthetic dose was increased, causing death. Following explantation of the livers, the liver flukes were isolated from the bile ducts and liver tissue.

*Fasciola hepatica* metacercaria culture.

AT the W. Stefaski Institute of Parasitology POLISH ACADEMY OF SCIENCES, the full developmental cycle of *Fasciola hepatica* is maintained. Thus we have access to a culture of the intermediate host of the parasite, the snail *Galba truncatula* and their primary food, algae of the genus *Oscillatoria.*

Eggs were isolated from live liver flukes originating from calf livers, and wre then stored in oligovcene water at 4˚C. On the day of the planned infection of the snail, the eggs were placed underneath a strong light source for a length of time, which causes the hatching of the miracidia. The *Galba truncatula* snails were infected by placing them in shallow plastic wells along with 3 miracidia for long enough for the miracidia to penetrate the tissues of the host. After around 90 days of culturing, metacercaria were collected from the surface of the water and the bottom side of the lids of the rearing dishes.

On the day of infecting the rats, metacercaria from different snails were collected into one test tube, from which the number of metacercaria needed to infect an individual were counted out and separated into another test tube.

Collection and storage of sera.

The blood collected was left for 2 hours at 37˚C. After that time, it was placed into 4˚C. The following day, the blood was centrifuged in a tabletop centrifuge and the clean serum was collected into Eppendorf tubes. The serum was stored at -70˚C until the performance of the serological tests.

Oral vaccination of both sexes of rats of the Sprague-Dawley strain with the cysteine protease of the liver fluke in inclusion bodies

Experiment I

Individual groups of animals were immunised with proteins, which were expressed in E. *coli* bacteria in the form of inclusion bodies.

Group 1 - immunised orally with the protein of the catalytic domain of the *Fasciola hepatica* cysteine protease (PC) encoded by the sequence KAT_Nasza_MOT1_MOT2.

Group 2 - immunised orally with the protein precursor of *Fasciola hepatica* cysteine protease (PC) (both the leading and catalytic domains) encoded by the sequence Cala_Mot_N
Group 3 - infection control; treated orally with proteins from lysed, untransformed bacteria
Group 4 - physiological control.

In the first experiment, the following treatments were administered orally to the rats:

Group 1 - 300 micrograms proteins of the catalytic domain of the cysteine protease (PC) of *Fasciola hepatica* (coded by the sequence KAT_Nasza_MOT1_MOT2) in 0.9% NaCl;
Group 2 - 300 micrograms of the precursor protein (both the leading and catalytic domains) PC of *F. hepatica* (coded by the sequence Cala_Mot_N) in 0.9% NaCl;
Group 3 - 300 micrograms bacterial lysate proteins (*E. coli*), not transformed, in 0.9% NaCl
Group 4 - 250 microlitres of 0.9% NaCl

The volume of the suspension administered to one animal was 250ml. 28 days later, rats in individual groups were given a second oral dose of an identical preparation, but containing 100 micrograms of protein. 28 following the second individual rats were infected orally with 30 metacercaria of *Fasciola hepatica.*
35 days later, a large volume of blood was collected. The rats were put down, a dissection was carried out and the livers were analysed for the presence of the liver flukes.
Experiments IIa and II b were carried out to ascertain whether it is possible to increase the protectiveness of the vaccine by a modification of the antigen.
In experiment IIa, individual groups of animals were immunised with proteins, which were expressed in *E. coli* bacteria in the form of inclusion bodies.

Group 1 - immunised orally with the construct: HBV core domain::catalytic domain of the liver fluke cysteine protease, encoded by the sequence Moty_Cat_Ckrd.
Group 2 - physiological control

In experiment IIb individual groups of animals were immunised with proteins, which were expressed in E. *coli* bacteria in the form of inclusion bodies.

Group 1 - immunised orally with the construct: HBV core domain::catalytic domain of the liver fluke cysteine protease, joined by a glycine bridge, encoded by the sequence CWekGlyKatwPIG.
Group 2 - physiological control

The course of the experiments was identical to that described above.

Determination of the level of protection

The level of protection in vaccinated groups was counted by comparing the average number of liver flukes present in the liver of a vaccinated animal to the average number of liver flukes in an individual from a control group, which orally received lysate from unrecombined E. *coli.*
The protection level was calculated according to the formula:

$$(1 - d/k) * 100\%,$$

where:

d - the average number of liver flukes present in an individual from the group vaccinated with the precursor (both the leading and catalytic domains, the sequence Cala_Mot_N) of cysteine protease or just the catalytic domain of this protease (the sequence KAT_Nasza_MOT1_MOT2).
k - the average number of liver flukes present in an individual from the control group, who was given unrecombined bacteria.

Haematological analysis methods.

20 microlitres of sampled blood were mixed with an anticoagulant and analysed automatically in the AutoCounter AC920 device (Swelab). The data obtained were the hematocrit (HCT), number of erythrocytes (RBC), average erythrocyte volume (MCV), haemoglobin content (HGB), average haemoglobin content per erythrocyte (MCH), number of platelets (PLT) and number of leucocytes (WBC).

Subpopulations of leucocytes were determined by counting the number of cells on microscope slides of blood smears using the Pappenheim method.

Immunoenzymatic assay (ELISA).

Rat sera collected during the experiment underwent an immunoenzymatic assay both individually, and pooled within the individual experimental groups.

Flat-bottomed ELISA microplates were coated overnight at 4˚C with excretion-secretion proteins of (ES) *Fasciola hepatica* at a concentration of 15 micrograms/ml in carbonate buffer (pH 9.6). 100 microlitres of the coating mixture were used per well. Next, the wells were washed three times in TBS (10 mM Tris, 0.15 M NaCl), with 0.05% Tween 40, pH 7.4. Non-specific binding was blocked by introducing 100 microlitres of 4% solution of skimmed milk in TBS into the wells, for two hours at room temperature. Next, a double wash was applied: TBS and TBS + Tween. Sera studied were then introduced into the wells, diluted 1:100 in a 2% skimmed milk solution in TBS. Each trial was repeated two or three times. The incubation lasted for 30 min. at 37˚C. Subsequently, there were 4 washings in TBS + Tween. Next, a serum recognizing particular types of rat antibodies was added. In the case of IgG, IgM and IgE these were sera tagged with horseradish peroxidase (HRP), from the sheep (IgG, IgM) or mouse (IgE). In the case of anti-IgA we only had an untagged serum from the mouse, so we had to add a third, ovine anti-mouse IgG serum tagged with HRP. All of the sera were obtained from Serotec and were diluted for various tests, according to the manufacturer's instructions).

The enzymatic reaction was carried out after a triple rinse in TBS + Tween, using 3,3',5,5' - tetramethylbenzene (TMB) as a substrate. The enzymatic reaction was halted after 30 minutes by adding 50 microlitres 2M of sulphuric acid, and optical density was studied using an automatic ELISA plate reader (Dynatech Laboratories) at a wavelength of 405 nm.

Results and Discusssion

Experiment I.

**Table 1. Protection level**

| Group | No. of rats | % protection | Statistical significance | Liver damage index |
|---|---|---|---|---|
| 1 | 8 | 79 | P<0.001 | 1,1 |
| 2 | 8 | 73 | P<0.05 | 1,3 |
| 3 | 8 | 0 | | 3,56 |

The results of the parasitological experiment show that a double oral administration of recombined cysteine protease protein of *Fasciola hepatica* causes a very high level of immunity against the infections with this flatworm. From the analysis of liver damage, it is shown that in the case of rats immunised against PC, only a few liver flukes have undertaken their migration through this organ.

To date there have been no mentions in specialist literature regarding the immunisation against an *F. hepatica* invasion through the oral administration of liver fluke antigens in the form of inclusion bodies.

Haematological parameters.

No significant differences were found in the number of platelets. The number of erythrocytes fell insignificantly two weeks following the infection, but the haemoatocrit did not change. The total number of leukocytes underwent a slight fluctuation, whereas the numbers of cells in individual subpopulations changed.

The largest changes were exhibited by the eosinophyls in group 3. A slight increase in these cells occurs already 14 days after the dose, whereas on day 28 post infection a serious eosinophylia develops, which maintains its level 42 days post invasion. The increase in the number of eosinophyls in groups 1 and 2 was insignificant, which may reaffirm that the invasion in the vaccinated rats was very mild.

Serological assay results.

Results obtained by the ELISA method.

Results of the assays for the level of antibodies of the classes IgG1, IgG2a, IgG2b, IgE and IgA reacting with PC or ES of the liver fluke are presented in Figures 15-20.

The analysis of the ELISA tests indicates that rats vaccinated orally, reacted to the invasion with an antibody response dependent on Th2 lymphocytes. It is thought that the immunological response expressed by Th2 cell-dependent antibodies is a defensive characteristic against helminth invasions.

Experiment IIa

Table 2. Protection level

| **Group** | **L. szczurów** | **% protekcji** | **Istotnośé * statystyczna** | **Indeks uszkodzeń watroby** |
|---|---|---|---|---|
| 1 | 8 | 61.6 | P<0.05 | 1,4 |
| 2 | 8 | 0 | | 4,1 |

Experiment IIb

Table 3. Protection level

| Group | L. szczurów | % protekcji | Istotnośé * statystyczna | Indeks uszkodzeń watroby |
|---|---|---|---|---|
| 1 | 8 | 65.5 | P<0.05 | 1,3 |
| 2 | 8 | 0 | | 4,4 |
| *the average number of liver flukes in the vaccinated group vs. the average number of liver flukes in the vaccinated group ("challenge" control) | | | | |

To summarise the results obtained in the present experiments, it should be strongly stressed that in an indisputable way that an oral vaccine based on the *Fasciola hepatica* cysteine protease protein in the form of inclusion bodies may be effective in preventing the invasions of this flatworm.

A great advantage and novelty of such a vaccine is the possibility of administering it in the form of a suspension, along with drinking water.

## Claims

**1.** Inclusion bodies containing a polypeptide containing the amino-acid sequence of the sequence of the liver fluke cysteine protease, possibly fused to the amino-acid sequence of the HBV core protein, for use as an oral vaccine against the liver fluke.

**2.** Inclusion bodies according to Claim 1, **characterised in that** they are produced in *E.coli.*

3. Inclusion bodies according to Claim 1, **characterised in that** the polypeptide contains one of the following sequences:

- the sequence of the catalytic domain of the liver fluke cysteine protease fused to the sequence of the HBV core protein,
- the sequence of the leading domain of the liver fluke cysteine protease fused to the sequence of the HBV core protein,
- the sequence of the catalytic domain of the liver fluke cysteine protease fused to the sequence of the HBV core protein and glycine residues, forming a glycine bridge structure,
- the sequence of the catalytic domain of the liver fluke cysteine protease,
- the sequence of the precursor protein of the liver fluke cysteine protease encompassing the catalytic and leading domains.

4. Inclusion bodies according to Claim 1, **characterised in that** the polypeptide contains one of the sequences encoded by the sequences presented in Figures 7 to 15.

5. Use of a polypeptide in the form of inclusion bodies containing the amino-acid sequence of the sequence of the liver fluke cysteine protease, possibly fused to the amino-acid sequence of the HBV core protein, in the preparation of a vaccine against the liver fluke.

6. Use according to Claim 5, **characterised in that** the polypeptide is produced in a genetically modified microorganism.

7. Use according to Claim 5, **characterised in that** the polypeptide contains one of the following sequences:

- the sequence of the catalytic domain of the liver fluke cysteine protease fused to the sequence of the HBV core protein,
- the sequence of the leading domain of the liver fluke cysteine protease fused to the sequence of the HBV core protein,
- the sequence of the catalytic domain of the liver fluke cysteine protease fused to the sequence of the HBV core protein and glycine residues, forming a glycine bridge structure,
- the sequence of the catalytic domain of the liver fluke cysteine protease,
- the sequence of the precursor protein of the liver fluke cysteine protease encompassing the catalytic and leading domains.

8. Use according to Claim 5, **characterised in that** the polypeptide contains one of the sequences encoded by the sequences presented in Figures 7 to 15.

9. An oral vaccine against the liver fluke containing the antigen and possibly pharmaceutically acceptable carrier and/or adjuvant, **characterised in that** as the antigen it contains a polypeptide in the form of inclusion bodies containing the amino-acid sequence of the sequence of the liver fluke cysteine protease, possibly fused to the amino-acid sequence of the HBV core protein.

10. A vaccine according to Claim 9, **characterised in that** the polypeptide is produced in a genetically modified microorganism.

11. A vaccine according to Claim 9, **characterised in that** the polypeptide contains one of the following sequences:

- the sequence of the catalytic domain of the liver fluke cysteine protease fused to the sequence of the HBV core protein,
- the sequence of the leading domain of the liver fluke cysteine protease fused to the sequence of the HBV core protein,
- the sequence of the catalytic domain of the liver fluke cysteine protease fused to the sequence of the HBV core protein and glycine residues, forming a glycine bridge structure,
- the sequence of the catalytic domain of the liver fluke cysteine protease,
- the sequence of the precursor protein of the liver fluke cysteine protease encompassing the catalytic and leading domains.

12. A vaccine according to Claim 9, **characterised in that** the polypeptide contains one of the sequences encoded by the sequences presented in Figures 7 to 15.

## Patentansprüche

1. Einschlußkörper, enthaltend ein Polypeptid, enthaltend die Aminosäuresequenz der Sequenz der Leberegel-Cysteinprotease, gegebenenfalls fusioniert an die Aminosäuresequenz des HBV-Kernproteins, zur Verwendung als ein oraler Impfstoff gegen den Leberegel.

2. Einschlußkörper nach Anspruch 1, **dadurch gekennzeichnet, daß** diese in *E.coli* hergestellt werden.

3. Einschlußkörper nach Anspruch 1, **dadurch gekennzeichnet, daß** das Polypeptid eine der folgenden Sequenzen enthält:

   - die Sequenz der katalytischen Domäne der Leberegel-Cysteinprotease, fusioniert an die Sequenz des HBV-Kernproteins,
   - die Sequenz der Leitdomäne der Leberegel-Cysteinprotease, fusioniert an die Sequenz des HBV-Kernproteins,
   - die Sequenz der katalytischen Domäne der Leberegel-Cysteinprotease, fusioniert an die Sequenz des HBV-Kernproteins und Glyzinreste, die eine Glyzin-Brückenstruktur ausbilden,
   - die Sequenz der katalytischen Domäne der Leberegel-Cysteinprotease,
   - die Sequenz des Vorläuferproteins der Leberegel-Cysteinprotease, umfassend die katalytische und Leit-Domänen.

4. Einschlußkörper nach Anspruch 1, **dadurch gekennzeichnet, daß** das Polypeptid eine der Sequenzen enthält, die von den Sequenzen kodiert wird, die in den Figuren 7 bis 15 dargestellt sind.

5. Verwendung eines Polypeptids in Form von Einschlußkörpern, enthaltend die Aminosäuresequenz der Sequenz der Leberegel-Cysteinprotease, gegebenenfalls fusioniert an die Aminosäuresequenz des HBV-Kernproteins zur Herstellung eines Impfstoffs gegen den Leberegel.

6. Verwendung nach Anspruch 5, **dadurch gekennzeichnet, daß** das Polypeptid in einem genetisch modifizierten Mikroorganismus hergestellt wird.

7. Verwendung nach Anspruch 5, **dadurch gekennzeichnet, daß** das Polypeptid eine der folgenden Sequenzen enthält:

   - die Sequenz der katalytischen Domäne der Leberegel-Cysteinprotease, fusioniert an die Sequenz des HBV-Kernproteins,
   - die Sequenz der Leitdomäne der Leberegel-Cysteinprotease, fusioniert an die Sequenz des HBV-Kernproteins,
   - die Sequenz der katalytischen Domäne der Leberegel-Cysteinprotease, fusioniert an die Sequenz des HBV-Kernproteins und Glyzinreste, die eine Glyzin-Brückenstruktur ausbilden,
   - die Sequenz der katalytischen Domäne der Leberegel-Cysteinprotease,
   - die Sequenz des Vorläuferproteins der Leberegel-Cysteinprotease, umfassend die katalytische und Leit-Domänen.

8. Verwendung nach Anspruch 5, **dadurch gekennzeichnet, daß** das Polypeptid eine der Sequenzen enthält, die von den Sequenzen kodiert wird, die in den Figuren 7 bis 15 dargestellt sind.

9. Oraler Impfstoff gegen den Leberegel, enthaltend das Antigen und gegebenenfalls pharmazeutisch akzeptablen Träger und/oder Adjuvans, **dadurch gekennzeichnet, daß** er als das Antigen ein Polypeptid in der Form von Einschlußkörpern, enthaltend die Aminosäuresequenz der Sequenz der Leberegel-Cysteinprotease, gegebenenfalls fusioniert an die Aminosäuresequenz des HBV-Kernproteins, enthält.

10. Impfstoff nach Anspruch 9, **dadurch gekennzeichnet, daß** das Polypeptid in einem genetisch modifizierten Mikroorganismus hergestellt wird.

11. Impfstoff nach Anspruch 9, **dadurch gekennzeichnet, daß** das Polypeptid eine der folgenden Sequenzen enthält:

- die Sequenz der katalytischen Domäne der Leberegel-Cysteinprotease, fusioniert an die Sequenz des HBV-Kernproteins,
- die Sequenz der Leitdomäne der Leberegel-Cysteinprotease, fusioniert an die Sequenz des HBV-Kernproteins,
- die Sequenz der katalytischen Domäne der Leberegel-Cysteinprotease, fusioniert an die Sequenz des HBV-Kernproteins und Glyzinreste, die eine Glyzin-Brückenstruktur ausbilden,
- die Sequenz der katalytischen Domäne der Leberegel-Cysteinprotease,
- die Sequenz des Vorläuferproteins der Leberegel-Cysteinprotease, umfassend die katalytische und Leit-Domänen.

**12.** Impfstoff nach Anspruch 9, **dadurch gekennzeichnet, daß** das Polypeptid eine der Sequenzen enthält, die von den Sequenzen kodiert wird, die in den Figuren 7 bis 15 dargestellt sind.

**Revendications**

**1.** Corps d'inclusion contenant un polypeptide contenant la séquence d'acides aminés de la séquence de la cystéine protéase de la douve, éventuellement fusionnée à la séquence d'acides aminés de la protéine coeur de HBV, pour une utilisation en tant que vaccin à usage oral contre la douve.

**2.** Corps d'inclusion selon la revendication 1, **caractérisés en ce qu'**ils sont produits dans *E. coli.*

**3.** Corps d'inclusion selon la revendication 1, **caractérisés en ce que** le polypeptide contient l'une des séquences suivantes :

- la séquence du domaine catalytique de la cystéine protéase de la douve fusionnée à la séquence de la protéine coeur de HBV,
- la séquence du domaine de tête de la cystéine protéase de la douve fusionnée à la séquence de la protéine coeur de HBV,
- la séquence du domaine catalytique de la cystéine protéase de la douve fusionnée à la séquence de la protéine coeur de HBV et à des résidus de glycine, formant une structure de pont de glycine,
- la séquence du domaine catalytique de la cystéine protéase de la douve,
- la séquence de la protéine précurseur de la cystéine protéase de la douve englobant les domaines catalytique et de tête.

**4.** Corps d'inclusion selon la revendication 1, **caractérisés en ce que** le polypeptide contient l'une des séquences codées par les séquences présentées sur les Figures 7 à 15.

**5.** Utilisation d'un polypeptide sous la forme de corps d'inclusion contenant la séquence d'acides aminés de la séquence de la cystéine protéase de la douve, éventuellement fusionnée à la séquence d'acides aminés de la protéine coeur de HBV, dans la préparation d'un vaccin contre la douve.

**6.** Utilisation selon la revendication 5, **caractérisée en ce que** le polypeptide est produit dans un microorganisme génétiquement modifié.

**7.** Utilisation selon la revendication 5, **caractérisée en ce que** le polypeptide contient l'une des séquences suivantes :

- la séquence du domaine catalytique de la cystéine protéase de la douve fusionnée à la séquence de la protéine coeur de HBV,
- la séquence du domaine de tête de la cystéine protéase de la douve fusionnée à la séquence de la protéine coeur de HBV,
- la séquence du domaine catalytique de la cystéine protéase de la douve fusionnée à la séquence de la protéine coeur de HBV et à des résidus de glycine, formant une structure de pont de glycine,
- la séquence du domaine catalytique de la cystéine protéase de la douve,
- la séquence de la protéine précurseur de la cystéine protéase de la douve englobant les domaines catalytique et de tête.

**8.** Utilisation selon la revendication 5, **caractérisée en ce que** le polypeptide contient l'une des séquences codées par les séquences présentées sur les Figures 7 à 15.

**9.** Vaccin à usage oral contre la douve, contenant l'antigène et éventuellement un véhicule et/ou un adjuvant acceptables en pharmacie, **caractérisé en ce qu'**à titre d'antigène, il contient un polypeptide sous la forme de corps d'inclusion contenant la séquence d'acides aminés de la séquence de la cystéine protéase de la douve, éventuellement fusionnée à la séquence d'acides aminés de la protéine coeur de HBV.

**10.** Vaccin selon la revendication 9, **caractérisé en ce que** le polypeptide est produit dans un microorganisme génétiquement modifié.

**11.** Vaccin selon la revendication 9, **caractérisé en ce que** le polypeptide contient l'une des séquences suivantes :

- la séquence du domaine catalytique de la cystéine protéase de la douve fusionnée à la séquence de la protéine de coeur HBV,
- la séquence du domaine de tête de la cystéine protéase de la douve fusionnée à la séquence de la protéine coeur de HBV,
- la séquence du domaine catalytique de la cystéine protéase de la douve fusionnée à la séquence de la protéine coeur de HBV et à des résidus de glycine, formant une structure de pont de glycine,
- la séquence du domaine catalytique de la cystéine protéase de la douve,
- la séquence de la protéine précurseur de la cystéine protéase de la douve englobant les domaines catalytique et de tête.

**12.** Vaccin selon la revendication 9, **caractérisé en ce que** le polypeptide contient l'une des séquences codées par les séquences présentées sur les Figures 7 à 15.

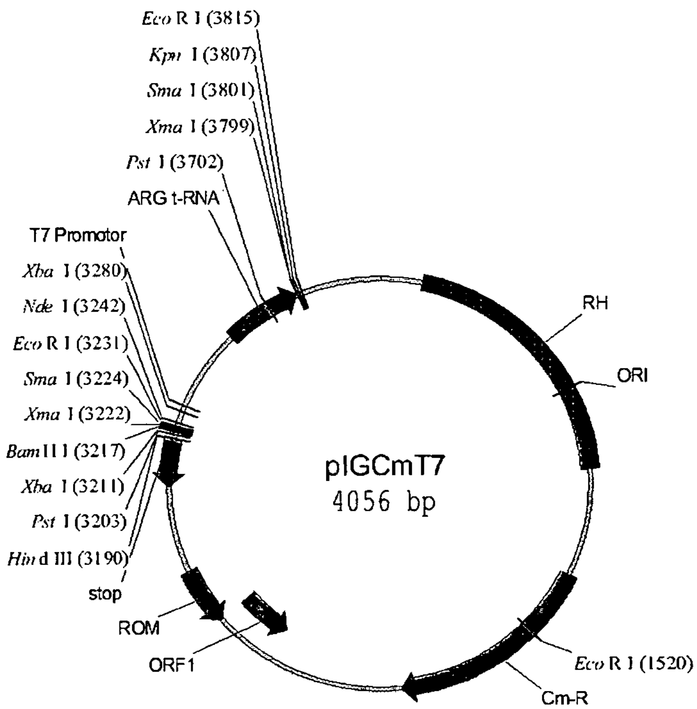
Eco R I (3815)
Kpn I (3807)
Sma I (3801)
Xma I (3799)
Pst I (3702)
ARG t-RNA
T7 Promotor
Xba I (3280)
Nde I (3242)
Eco R I (3231)
Sma I (3224)
Xma I (3222)
BamH I (3217)
Xba I (3211)
Pst I (3203)
Hin d III (3190)
stop
ROM
ORF1
RH
ORI
Eco R I (1520)
Cm-R
pIGCmT7
4056 bp

Fig. 1

GGATCCATGTGGTTCTTCGTATTAGCCGTCCTCACAGTCGGAGTGCTTGGCTCGAATGATGATTTGTGGCATCAGTGGAAGCGAATGTACAACAA
TGAATACAATGGGGCTGACGATCAGCACAGACGAAATATTTGGGAAGAGAATGTGAAACATATCCAAGAACATAACCTACGTCACGATCTCGGCC
TCGTCACCTACACATTGGGATTGAACCAATTCACGGATATGACATTCGAGGAATTCAAGGCCAAATATCTAACAGAAATGTCACGCGCGTCCGAT
ATACTCTCACACGGTGTCCCGTATGAGACGAACAATCGTGCCGTACCCGACAAAATTGACTGGCGTGAATCTGGTTATGTGACGGAGGTGAAAGA
TCAGGGAAACTGTGGTTCCTGTTGGGCATTCTCAACAACCGGTACTATGGAGGGACAGTATATGAAAAACGAAAGAACTAGTATTTCATTCTCTG
AGCAACAACTGGTCGATTGTAGCGGTCCTTGGGGAAATAATGGTTGCAGTGGTGGATTGATGGAAAATGCTTACCAATATTTGAAACAATTTGGA
TTGGAAACCGAATCCTCTTATCCGTACACGGCTGTGGAAGGTCAGTGTCGATACAATAAGCAGTTAGGAGTTGCCAAAGTGACTGGCTACTACAC
TGTGCCTTCTGGCAGTGAGGTAGAATTGAAAAATCTAGTCGGTGCCGAAGGACCTGCCGCGGTCGCTGTGGATGTGGAATCTGACTTCATGATGT
ACAGGAGTGGTATTTATCAGAGCCAAACTTGTTCACCGCTTCGTGTGAATCATGCAGTCTTGGCTGTCGGTTACGGAACACAGGGTGGTACTGAC
TATTGGATTGTGAAAAATAGTTGGGGATTGTCGTGGGGTGAGCGCGGTTACATTCGAATGGCTAGGAATCGAGGTAACATGTGTGGAATTGCTTC
GCTGGCCAGTCTCCCGATTGGCACGATTTCCGTGACTCGAG

**Fig. 2**

1 2

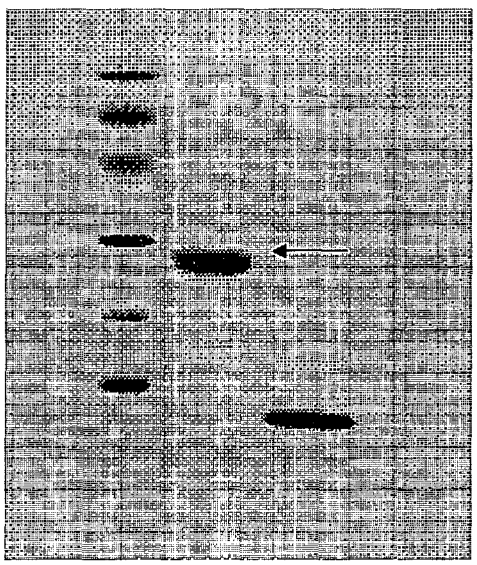

Fig. 3

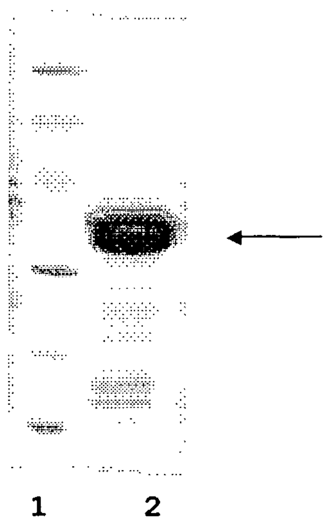

Fig. 4

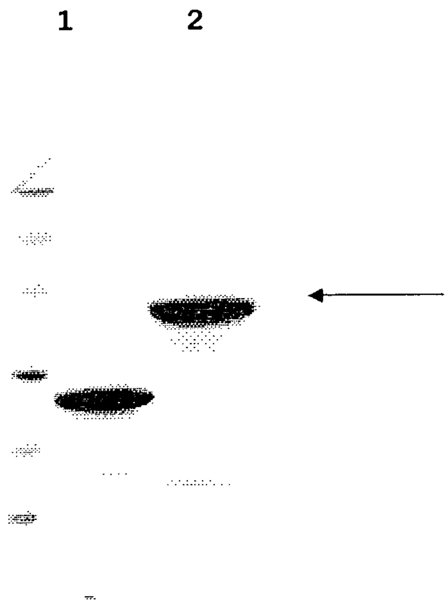


Fig. 5

1 2

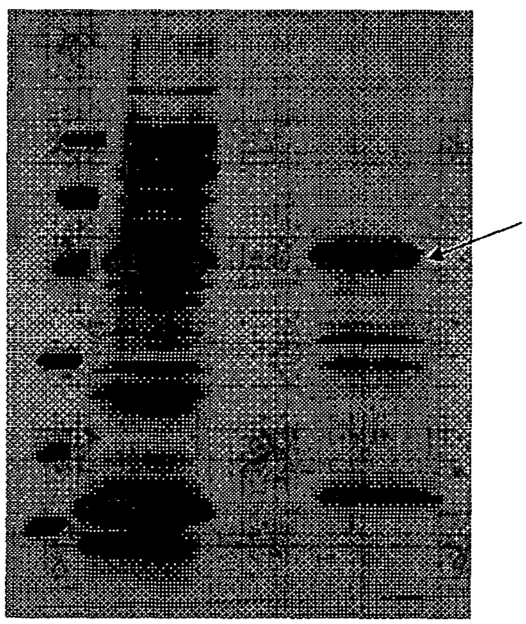

Fig. 6

*Nde*I

cat**atg**gatatcga tccttataaa gaatttggag ctactgtgga gttactctcg tttttgcctt ctgacttctt
tccttcagta cgagatcttc tggataccgc ctcagctctc tatcgggaag ccttagagtc tcctgagcat
tgttcacctc accatactgc actcaggcaa gcaattcttt gctgggggga actaatgact ctagctacct
gggtgggtgt taatctcgag gatcaaggtg gtggaggttc tggtggagga ggccaaggag gcgctgtacc
cgacaaaatt gactggcgtg aatctggtta tgtgacggag gtgaaagatc agggaaactg tggttcctgt
tgggcattct caacaaccgg tactatggag ggacagtata tgaaaaacga aagaactagt atttcattct
ctgagcaaca actggtcgat tgtagcggtc cttggggaaa taatggttgc agtggtggat tgatggaaaa
tgcttaccaa tatttgaaac aatttggatt ggaaaccgaa tcctcttatc cgtacacggc tgtggaaggt
cagtgtcgat acaataagca gttaggagtt gccaaagtga ctggctacta cactgtgcct tctggcagtg
aggtagaatt gaaaaatcta gtcggtgccg aaggacctgc cgcggtcgct gtggatgtgg aatctgactt
catgatgtac aggagtggta tttatcagag ccaaacttgt tcaccgcttc gtgtgaatca tgcagtcttg
gctgtcggtt acggaacaca gggtggtact gactattgga ttgtgaaaaa tagttgggga ttgtcgtggg
gtgagcgcgg ttacattcga atggctagga atcgaggtaa catgtgtgga attgcttcgc tggccagtct
cccgattggc acgatttccg gcatcgatgg ccaaggaggc caaggaggag gtggttcagg cggtggtgga
tctagggacc tagttgttag ttatgtcaac actaatatgg gcttaaagtt caggcaactt ttgtggtttc
acatttcttg tctcactttt ggaagagaaa cggtcataga gtatttggtg tctttcggag tgtggattcg
cactcctcca gcttatagac caccaaatgc ccctatctta tcaacacttc cggagactac tgttgtttaa
gctt
*Hind*III

**Fig. 7**

*Nde*I
cat**atg**gata tcgatcctta taaagaattt ggagctactg tggagttact ctcgtttttg ccttctgact
tctttccttc agtacgagat cttctggata ccgcctcagc tctctatcgg gaagccttag agtctcctga
gcattgttca cctcaccata ctgcactcag gcaagcaatt ctttgctggg gggaactaat gactctagct
acctgggtgg gtgttaatct cgaggatcaa ggtggtggag gttctggtgg aggaggccaa ggaggcgctg
tacccgacaa aattgactgg cgtgaatctg gttatgtgac ggaggtgaaa gatcagggaa actgtggttc
ctgttgggca ttctcaacaa ccggtactat ggagggacag tatatgaaaa acgaaagaac tagtatttca
ttctctgagc aacaactggt cgattgtagc ggtccttggg gaaataatgg ttgcagtggt ggattgatgg
aaaatgctta ccaatatttg aaacaatttg gattggaaac cgaatcctct tatccgtaca cggctgtgga
aggtcagtgt cgatacaata agcagttagg agttgccaaa gtgactggct actacactgt gccttctggc
agtgaggtag aattgaaaaa tctagtcggt gccgaaggac ctgccgcggt cgctgtggat gtggaatctg
acttcatgat gtacaggagt ggtatttatc agagccaaac ttgttcaccg cttcgtgtga atcatgcagt
cttggctgtc ggttacggaa cacagggtgg tactgactat tggattgtga aaaatagttg gggattgtcg
tggggtgagc gcggttacat tcgaatggct aggaatcgag gtaacatgtg tggaattgct tcgctggcca
gtctcccgat tggcacgatt tccggcatcg atggccaagg aggccaagga ggaggtggtt caggcggtgg
tggatctagg gacctagttg ttagttatgt caacactaat atgggcttaa agttcaggca actttgtgg
tttcacattt cttgtctcac ttttggaaga gaaacggtca tagagtattt ggtgtctttc ggagtgtgga
ttcgcactcc tccagcttat agaccaccaa atgcccctat cttatcaaca cttccggaga ctactgttgt
t**taa**gctt
*Hind*III

**Fig. 8**

*Bam*HI

ggatccat**at** **g**gctgtaccc gacaaaattg actggcgtga atctggttat gtgacggagg tgaaagatca
gggaaactgt ggttcctgtt gggcattctc aacaaccggt actatggagg gacagtatat gaaaaacgaa
agaactagta tttcattctc tgagcaacaa ctggtcgatt gtagcggtcc ttggggaaat aatggttgca
gtggtggatt gatggaaaat gcttaccaat atttgaaaca atttggattg gaaaccgaat cctcttatcc
gtacacggct gtggaaggtc agtgtcgata caataagcag ttaggagttg ccaaagtgac tggctactac
actgtgcctt ctggcagtga ggtagaattg aaaaatctag tcggtgccga aggacctgcc gcggtcgctg
tggatgtgga atctgacttc atgatgtaca ggagtggtat ttatcagagc caaacttgtt caccgcttcg
tgtgaatcat gcagtcttgg ctgtcggtta cggaacacag ggtggtactg actattggat tgtgaaaaat
agttggggat tgtcgtgggg tgagcgcggt tacattcgaa tggctaggaa tcgaggtaac atgtgtggaa
ttgcttcgct ggccagtctc ccgattggca cgatttccgg catcgatcct tataaagaat ttggagctac
tgtggagtta ctctcgtttt tgccttctga cttctttcct tcagtacgag atcttctaga taccgcctca
gctctctatc gggaagcctt agagtctcct gagcattgtt cacctcacca tactgcactc aggcaagcaa
ttctttgctg gggggaacta atgactctag ctacctgggt gggtgttaat ttggaagatc cagcatctag
ggacctagtt gttagttatg tcaacactaa tatgggctta aagttcaggc aacttttgtg gtttcacatt
tcttgtctca cttttggaag agaaacggtc atagagtatt tggtgtcttt cggagtgtgg attcgcactc
ctccagctta tagaccacca aatgccccta tcttatcaac acttccggag actactgttg tt**taa**aagct
tgagctc

*Sac*I

**Fig. 9**

*Bam*HI

ggatccat**at g**gctgtaccc gacaaaattg actggcgtga atctggttat gtgacggagg tgaaagatca
gggaaactgt ggttcctgtt gggcattctc aacaaccggt actatggagg gacagtatat gaaaaacgaa
agaactagta tttcattctc tgagcaacaa ctggtcgatt gtagcggtcc ttggggaaat aatggttgca
gtggtggatt gatggaaaat gcttaccaat atttgaaaca atttggattg gaaaccgaat cctcttatcc
gtacacggct gtggaaggtc agtgtcgata caataagcag ttaggagttg ccaaagtgac tggctactac
actgtgcctt ctggcagtga ggtagaattg aaaaatctag tcggtgccga aggacctgcc gcggtcgctg
tggatgtgga atctgacttc atgatgtaca ggagtggtat ttatcagagc caaacttgtt caccgcttcg
tgtgaatcat gcagtcttgg ctgtcggtta cggaacacag ggtggtactg actattggat tgtgaaaaat
agttggggat tgtcgtgggg tgagcgcggt tacattcgaa tggctaggaa tcgaggtaac atgtgtggaa
ttgcttcgct ggccagtctc ccgatggtgg cacgatttcc gggtagcggt agtgatatcg at**taa**gctt

*Hind*III

**Fig. 10**

*Nde*I

cat**atg**gctg tacccgacaa aattgactgg cgtgaatctg gttatgtgac ggaggtgaaa gatcagggaa
actgtggttc ctgttgggca ttctcaacaa ccggtactat ggagggacag tatatgaaaa acgaaagaac
tagtatttca ttctctgagc aacaactggt cgattgtagc ggtccttggg gaaataatgg ttgcagtggt
ggattgatgg aaaatgctta ccaatatttg aaacaatttg gattggaaac cgaatcctct tatccgtaca
cggctgtgga aggtcagtgt cgatacaata agcagttagg agttgccaaa gtgactggct actacactgt
gccttctggc agtgaggtag aattgaaaaa tctagtcggt gccgaaggac ctgccgcggt cgctgtggat
gtggaatctg acttcatgat gtacaggagt ggtatttatc agagccaaac ttgttcaccg cttcgtgtga
atcatgcagt cttggctgtc ggttacggaa cacagggtgg tactgactat tggattgtga aaaatagttg
gggattgtcg tggggtgagc gcggttacat tcgaatggct aggaatcgag gtaacatgtg tggaattgct
tcgctggcca gtctcccgat ggtggcacga tttccgggta gcggtagtga tatcgat**taa** gctt

*Hind*III

**Fig. 11**

*Bam*HI

ggatccat**at g**gctgtaccc gacaaaattg actggcgtga atctggttat gtgacggagg tgaaagatca
gggaaactgt ggttcctgtt gggcattctc aacaaccggt actatggagg gacagtatat gaaaaacgaa
agaactagta tttcattctc tgagcaacaa ctggtcgatt gtagtggtcc ttggggaaat aatggttgca
gtggtggatt gatggaaaat gcttaccaat atttgaaaca atttggattg gaaaccgaat cctcttatcc
gtacacggct gtggaaggtc agtgtcgata caataagcag ttaggagttg ccaaagtgac tggctactac
actgtgcctt ctggcagtga ggtagaattg aaaaatctag tcggtgccga aggacctgcc gcggtcgctg
tggatgtgga atctgacttc atgatgtaca ggagtggtat ttatcagagc caaacttgtt caccgcttcg
tgtgaatcat gcagtcttgg ctgtcggtta cggaacacag ggtggtactg actattggat tgtgaaaaat
agttggggat tgtcgtgggg tgagcgcggt tacattcgaa tggctaggaa tcgaggtaac atgtgtggaa
ttgcttcgct ggccagtctc ccgatggtgg cacgatttcc ggcatcgat**t aa**gctt

*Hind*III

**Fig. 12**

*Nde*I

cat**atg**gctg tacccgacaa aattgactgg cgtgaatctg gttatgtgac ggaggtgaaa gatcagggaa
actgtggttc ctgttgggca ttctcaacaa ccggtactat ggagggacag tatatgaaaa acgaaagaac
tagtatttca ttctctgagc aacaactggt cgattgtagt ggtccttggg gaaataatgg ttgcagtggt
ggattgatgg aaaatgctta ccaatatttg aaacaatttg gattggaaac cgaatcctct tatccgtaca
cggctgtgga aggtcagtgt cgatacaata agcagttagg agttgccaaa gtgactggct actacactgt
gccttctggc agtgaggtag aattgaaaaa tctagtcggt gccgaaggac ctgccgcggt cgctgtggat
gtggaatctg acttcatgat gtacaggagt ggtatttatc agagccaaac ttgttcaccg cttcgtgtga
atcatgcagt cttggctgtc ggttacggaa cacagggtgg tactgactat tggattgtga aaaatagttg
gggattgtcg tggggtgagc gcggttacat tcgaatggct aggaatcgag gtaacatgtg tggaattgct
tcgctggcca gtctcccgat ggtggcacga tttccggcat cgat**taa**gct t

*Hind*III

**Fig. 13**

*Nde*I
cat**atg**gctg tacccgacaa aattgactgg cgtgaatctg gttatgtgac ggaggtgaaa gatcagggaa
actgtggttc ctgttgggca ttctcaacaa ccggtactat ggagggacag tatatgaaaa acgaaagaac
tagtatttca ttctctgagc aacaactggt cgattgtagc ggtccttggg gaaataatgg ttgcagtggt
ggattgatgg aaaatgctta ccaatatttg aaacaatttg gattggaaac cgaatcctct tatccgtaca
cggctgtgga aggtcagtgt cgatacaata agcagttagg agttgccaaa gtgactggct actacactgt
gccttctggc agtgaggtag aattgaaaaa tctagtcggt gccgaaggac ctgccgcggt cgctgtggat
gtggaatctg acttcatgat gtacaggagt ggtatttatc agagccaaac ttgttcaccg cttcgtgtga
atcatgcagt cttggctgtc ggttacggaa cacagggtgg tactgactat tggattgtga aaaatagttg
gggattgtcg tggggtgagc gcggttacat tcgaatggct aggaatcgag gtaacatgtg tggaattgct
tcgctggcca gtctcccgat tggcacgatt tccggcatcg atccttataa agaatttgga gctactgtgg
agttactctc gtttttgcct tctgacttct ttccttcagt acgagatctt ctagataccg cctcagctct
ctatcgggaa gccttagagt ctcctgagca ttgttcacct caccatactg cactcaggca agcaattctt
tgctgggggg aactaatgac tctagctacc tgggtgggtg ttaatttgga agatccagca tctagggacc
tagttgttag ttatgtcaac actaatatgg gcttaaagtt caggcaactt ttgtggtttc acatttcttg
tctcactttt ggaagagaaa cggtcataga gtatttggtg tctttcggag tgtggattcg cactcctcca
gcttatagac caccaaatgc ccctatctta tcaacacttc cggagactac tgttgtt**taa** aagctt
*Hind*III

**Fig. 14**

*Nde*I

cat**atg**tcgaatgatgatttgtggcatcagtggaagcgaatgtacaacaatgaatacaatggggctgacgatcagcacaga
cgaaatatttgggaagagaatgtgaaacatatccaagaacataacctacgtcacgatctcggcctcgtcacctacacattg
ggattgaaccaattcacggatatgacattcgaggaattcaaggccaaatatctaacagaaatgtcacgcgcgtccgatata
ctctcacacggtgtcccgtatgagacgaacaatcgtgccgtacccgacaaaattgactggcgtgaatctggttatgtgacg
gaggtgaaagatcagggaaactgtggttcctgttgggcattctcaacaaccggtactatggagggacagtatatgaaaaac
gaaagaactagtatttcattctctgagcaacaactggtcgattgtagcggtccttggggaaataatggttgcagtggtgga
ttgatggaaaatgcttaccaatatttgaaacaatttggattggaaaccgaatcctcttatccgtacacggctgtggaaggt
cagtgtcgatacaataagcagttaggagttgccaaagtgactggctactacactgtgccttctggcagtgaggtagaattg
aaaaatctagtcggtgccgaaggacctgccgcggtcgctgtggatgtggaatctgacttcatgatgtacaggagtggtatt
tatcagagccaaacttgttcaccgcttcgtgtgaatcatgcagtcttggctgtcggttacggaacacagggtggtactgac
tattggattgtgaaaaatagttggggattgtcgtggggtgagcgcggttacattcgaatggctaggaatcgaggtaacatg
tgtggaattgcttcgctggccagtctcccgattggcacgatttccggcatcgat**taa**gctt

*Hind*III

**Fig. 15**

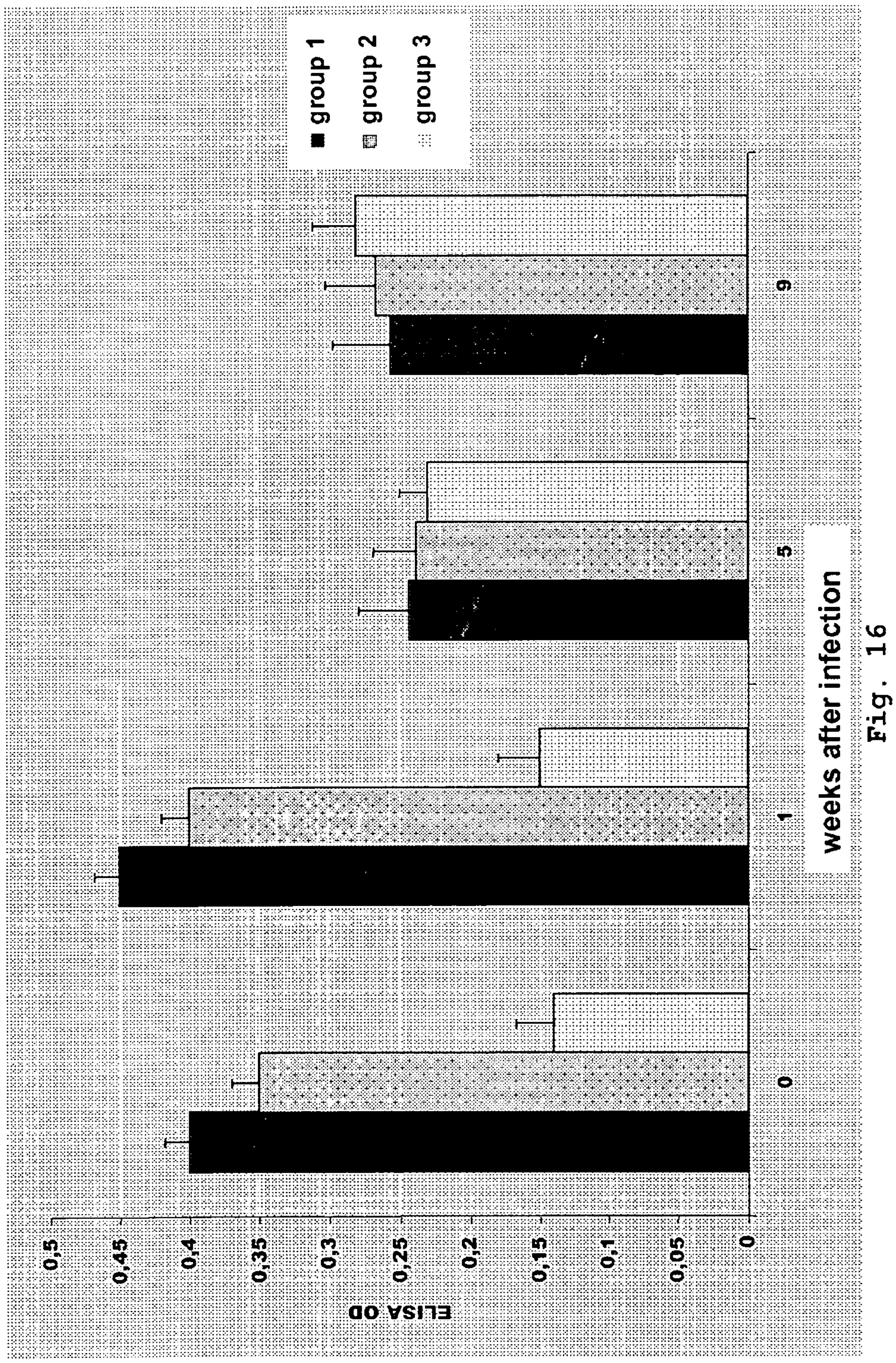
ELISA OD
0,5
0,45
0,4
0,35
0,3
0,25
0,2
0,15
0,1
0,05
0
0
1
5
9
weeks after infection
group 1
group 2
group 3

Fig. 16

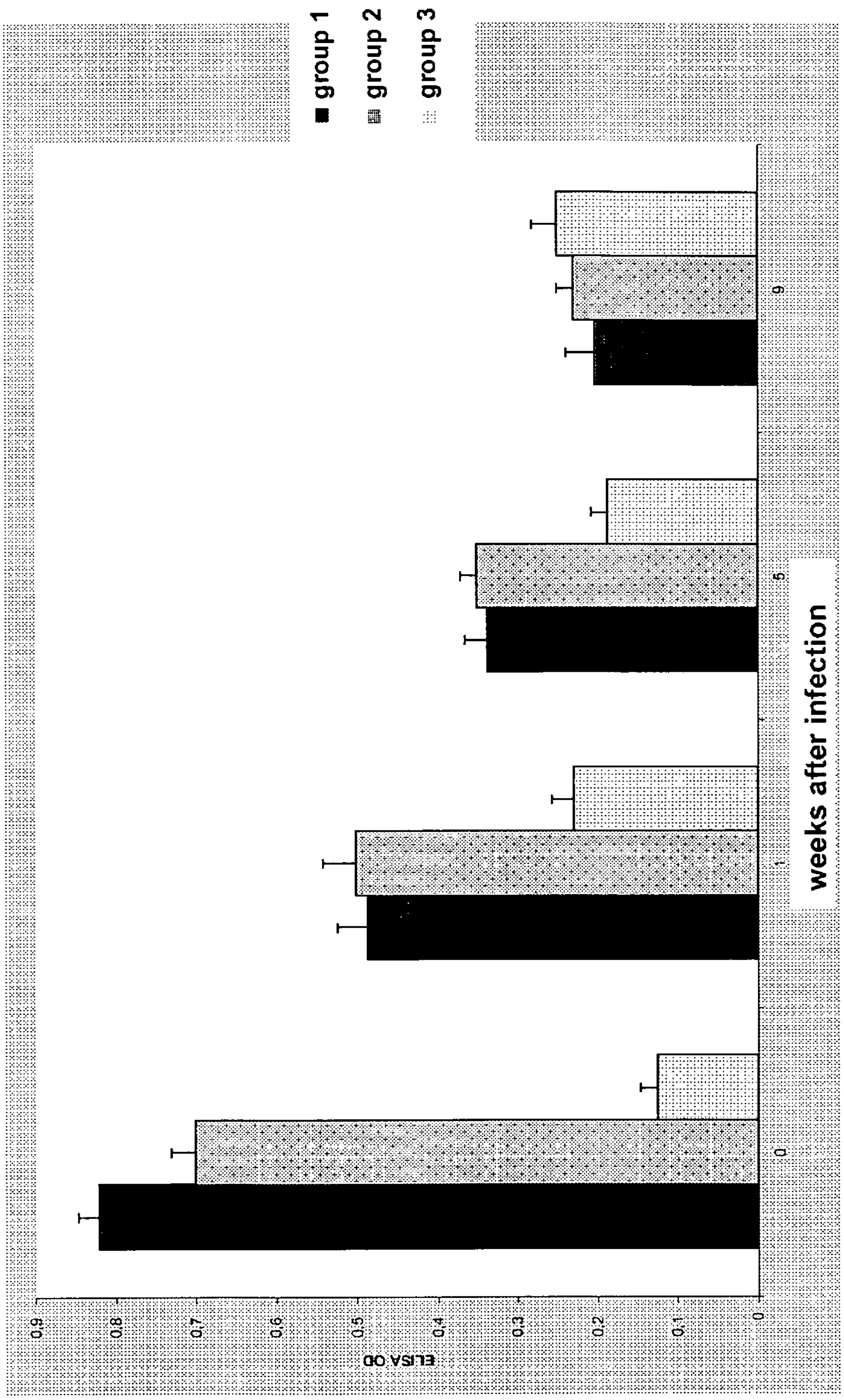
group 1
group 2
group 3
ELISA OD
0
0.1
0.2
0.3
0.4
0.5
0.6
0.7
0.8
0.9
0
1
5
9
weeks after infection

Fig. 17

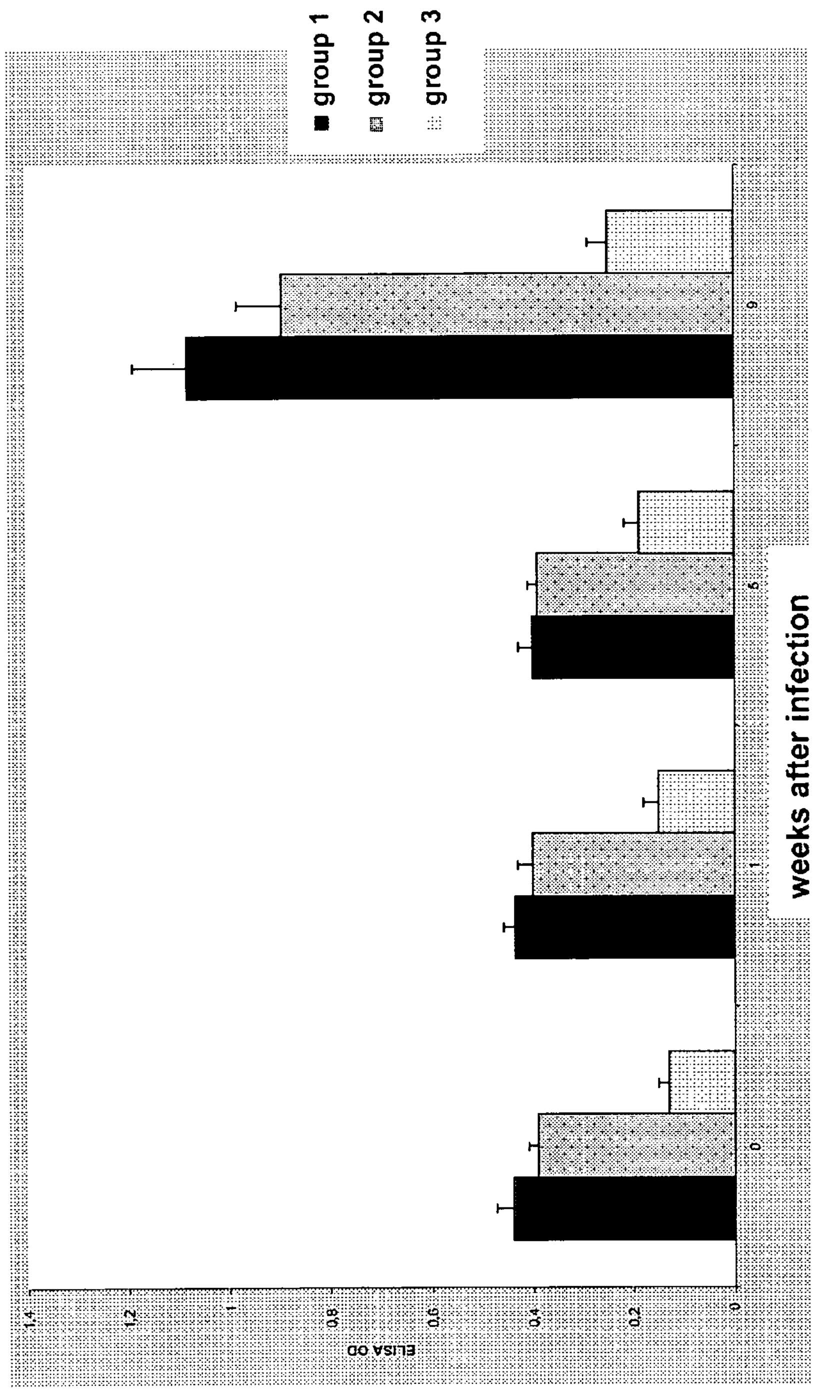
group 1
group 2
group 3
ELISA OD
1,4
1,2
1
0,8
0,6
0,4
0,2
0
0
1
5
9
weeks after infection

Fig. 18

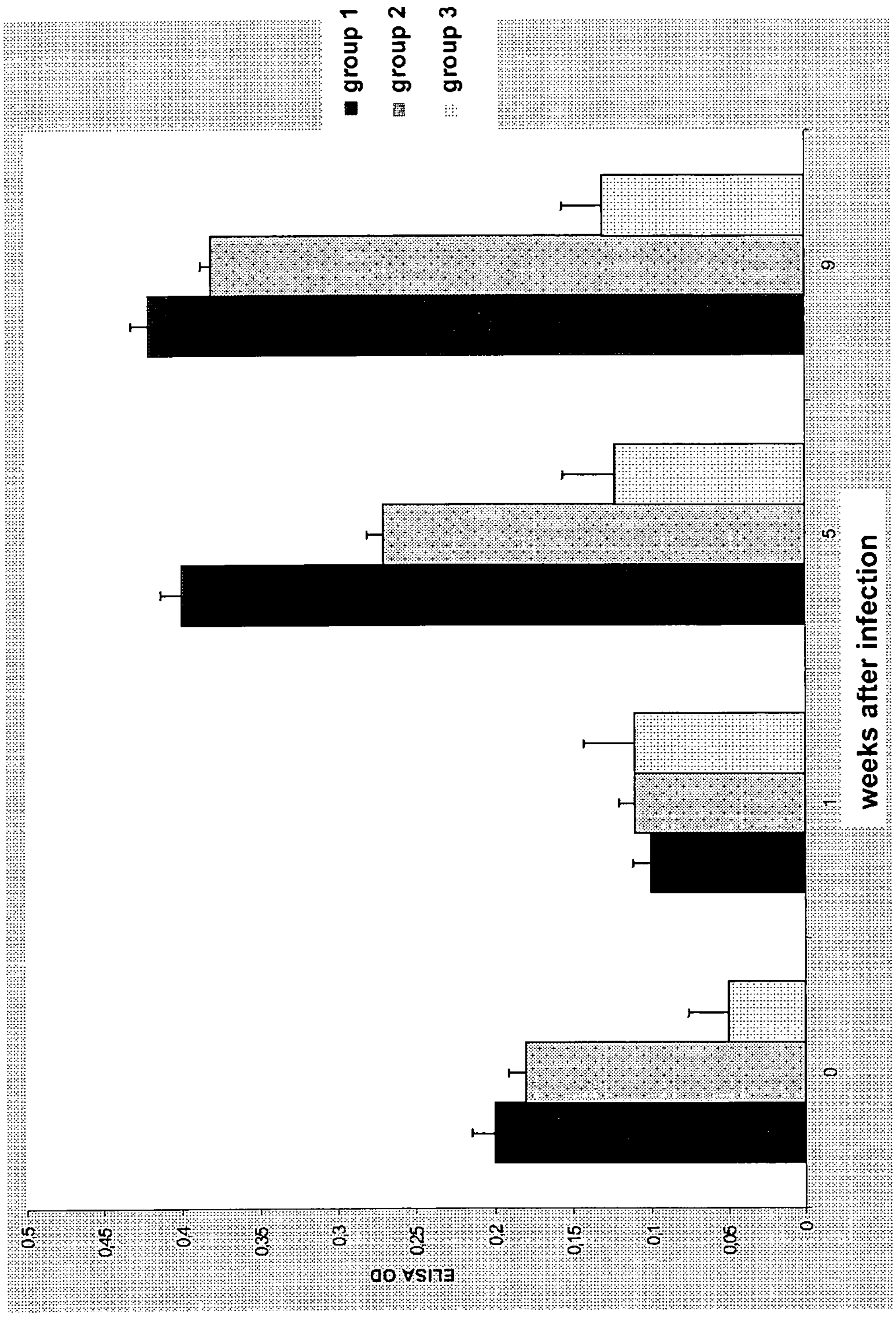
group 1
group 2
group 3
ELISA OD
0,5
0,45
0,4
0,35
0,3
0,25
0,2
0,15
0,1
0,05
0
0
1
5
9
weeks after infection

Fig. 19

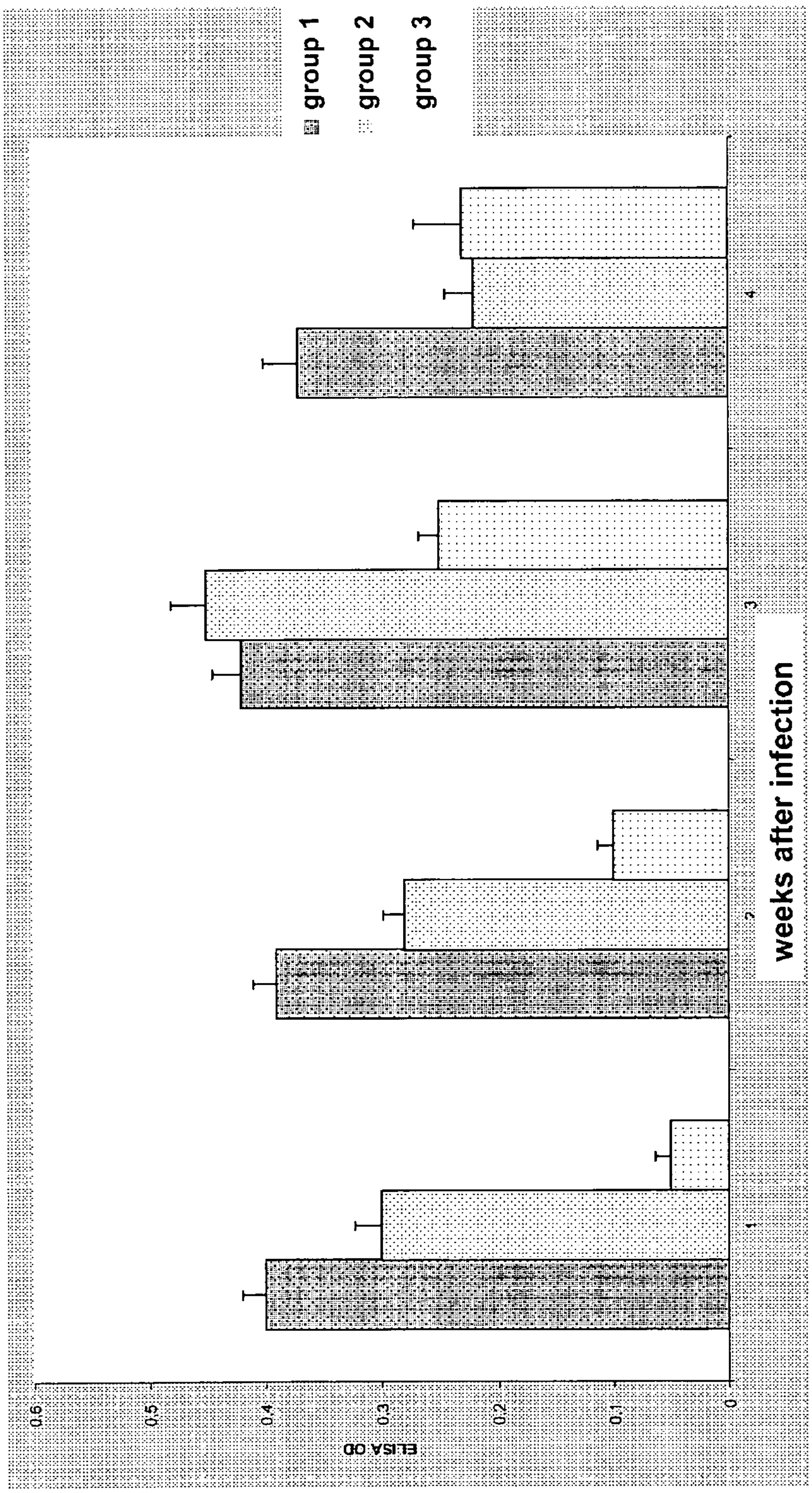
group 1
group 2
group 3
weeks after infection
ELISA OD
0.6
0.5
0.4
0.3
0.2
0.1
0
1
2
3
4

Fig. 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0037610 A
- US 5770214 A
- US 5811105 A
- US 5804194 A
- US 6290962 B
- PL 179149
- PL 174448
- WO 9832427 A
- US 6281345 B
- US 6265198 B
- US 6066503 A
- US 5885814 A
- US 6419923 B
- US 6365392 B
- US 5795768 A
- US 5792624 A
- US 5750391 A
- US 5691186 A
- US 5863775 A
- US 79840 A
- PL 358081
- PL 0300151 W

### Non-patent literature cited in the description


- **VAUGHAN et al.** *Aust. Vet. J.,* 1997, vol. 75 (11), 811-3
- **BORAY J.C.** NSW Agriculture Agfact. 1999
- **RICHTER L. ; KIPP B.B.** *Curr. Top Microbiol. Immunol.,* 1999, vol. 240, 159-176
- **MCGHEE J.R. et al.** Mucosal Immunology. Academic Press, 1999, 485-505
- **TACKET C.O. ; MASON H.S.** *Microbes and Infection,* 1999, vol. 1, 777-783
- **MAKELA P.H.** *FEMS Microbiology Reviews,* 2000, vol. 24, 9-20
- **RICHTER L. ; KIPP B.B.** *Curr. Top Microbiol. Immunol.,* 1999, vol. 240, 159-176
- **MIKIEWICZ D. et al.** *Plasmid,* 1997
- **KOFTA W ; MIESZCZANEK J ; PLUCIENNICZAK G ; WEDRYCHOWICZ H.** Successful DNA immunisation of rats against fasciolosis. *Vaccine,* 2000, vol. 18, 2985-2990
- **DYBCZYŃSKI I ; PHUCIENNICZAK A.** A protocol for DNA fragment extraction from polyacryloamide gels. *Biotechniques,* 1988, vol. 6, 924-926
- **J. SAMBROOK ; CHUNG C. T. ; MILLER R. H. et al.** A rapid and convenient method for the preparation and storage of competent bacterial cells. *Nucleic Acid. Res.,* 1988, vol. 16 (8